(19) Europäisches Patentamt / European Patent Office / Office européen des brevets

(11) **EP 2 675 904 B2**

(12) **NEW EUROPEAN PATENT SPECIFICATION**
After opposition procedure

(45) Date of publication and mention
of the opposition decision:
**14.08.2024 Bulletin 2024/33**

(45) Mention of the grant of the patent:
**14.02.2018 Bulletin 2018/07**

(21) Application number: **12704710.8**

(22) Date of filing: **17.02.2012**

(51) International Patent Classification (IPC):
**C12P 5/02** (2006.01)     **C12M 1/107** (2006.01)
**C10L 3/08** (2006.01)

(52) Cooperative Patent Classification (CPC):
**C12M 21/04; C10L 3/08; C12M 43/00; C12M 43/08;
C12P 5/023;** Y02E 50/30; Y02E 60/36;
Y02P 20/133; Y02P 20/59

(86) International application number:
**PCT/EP2012/000726**

(87) International publication number:
**WO 2012/110257 (23.08.2012 Gazette 2012/34)**

(54) **SYSTEM AND METHOD FOR STORING ENERGY IN THE FORM OF METHANE**

SYSTEM UND VERFAHREN ZUR SPEICHERUNG VON ENERGIE IN FORM VON METHAN

SYSTÈME ET PROCÉDÉ POUR STOCKER DE L'ÉNERGIE SOUS FORME DE MÉTHANE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.02.2011 EP 11154897**

(43) Date of publication of application:
**25.12.2013 Bulletin 2013/52**

(73) Proprietor: **Krajete GmbH
4061 Pasching (AT)**

(72) Inventor: **KRAJETE, Alexander
A-4020 Linz (AT)**

(74) Representative: **Lepthien, Sandra
Graf von Stosch
Patentanwaltsgesellschaft mbH
Prinzregentenstraße 22
80538 München (DE)**

(56) References cited:
**DE-A1- 102009 018 126     US-A1- 2009 130 734**

- **STERNER ET AL.: "Erneuerbares Methan",
SOLARZEITALTER, vol. 1, 2010, pages 51-58,
XP002676162**
- **SPECHT ET AL.: "Speicherung von Bioenergie
und erneuerbarem Strom im Erdgasnetz",
ERDÖL ERDGAS KOHLE, vol. 126, no. 10, 2010,
XP002676163**

EP 2 675 904 B2

**Description**

Against the background of decreasing reserves of fossil fuels and environmental awareness of global warming and carbon dioxide release, renewable energy sources are moving to the centre of attention. To date, renewable energy sources, i.e. energy from natural resources such as sunlight, wind, rain, tides and geothermal heat, cover about 13% of the global energy demand. Taking into consideration the criteria of sustainability and for achieving the targets of the Kyoto Protocol, it is indispensible to further expand the use of renewable energies. While most of the energy is consumed by a minority of the world's population, the energy demand of developing and emerging countries is increasing rapidly. However, while the world's energy demand is increasing, resources of fossil fuels are decreasing. It has been estimated that - even if consumption remains the same - oil reserves will be exhausted in 40-50 years, natural gas around 70 years and coal around 200 years. This estimation already includes the exploitation of oil and gas reserves which are currently not economical. Thus, energy prices will rise significantly, likely along with social and political conflicts, destruction of the environment and global warming. Beside the importance of fossil oil as energy source, it is also an important and essential resource for other industries such as chemistry, pharmaceutical industry and construction (~10% of the fossil oil). In the future, here also a new basis for most of the products is required.

However, currently only fossil and nuclear energy is available in storable form which allows for transport and always meets a temporarily fluctuating energy demand. In contrast, most renewable energy sources are intermittent, not transportable and principally dependent on the meteorological and geographical conditions. This is especially important for places such as for example northern Europe and America which have a high energy demand, but are - beside general daytime variations - confined to a seasonally fluctuating supply of renewable energies, such as solar or wind power. To fully replace fossil and nuclear power with energy from renewable and environmentally friendly sources in the future, energy from these sources will have to be converted into a storable and transportable form.

Most present approaches rely on renewable energy sources which very often suffer from a low energy recovery rate, fluctuating availability and limited efficiency. Another technical obstacle is a missing infrastructure: bioethanol for example is not suitable for many standard vehicle engines. Further, although the majority of energy consumers agree that fossil energy forms should be replaced by renewable energy sources, discussions regularly arise when agricultural area is required for biofuel production from biomass monoculture, such as for corn or sugarcane cultivation, or a vast area is selected for wind farm construction or hydroelectric dam projects, not even mentioned nuclear power plants which are sometimes also considered as an alternative to fossil-fuel power sources.

To date, various methods are available to convert energy from one energy form into another. However, the conversion rates are generally very low and for example in case of fossil fuel, only around 33% of the energy released reaches the end-user, the remaining around 67% of the energy is lost during conversion as heat or radiance, or in form of chemical substances which are produced during the conversion steps and which remain unused and are considered as waste.

One attempt in this direction is described in US 2010/0269498, wherein a system and method for the capture of the waste heat from generators is described. This "cogeneration" approach uses hydrogen as energy source. However, hydrogen has characteristics which limit its broad applicability, such as its explosiveness and tendency to leak from containers due to its small size. Moreover, hydrogen gas is less energy dense by volume when compared to other combustible fuels unless compressed or liquefied which further increases the risk of explosion and leakage. Also DE 10 2007 037 672 describes a method, wherein a method is described that uses renewable energy for the production of hydrogen which may later be used for the hydration of carbon dioxide by Fischer-Tropsch synthesis. Another obstacle of using hydrogen as energy carrier is the missing infrastructure.

Sterner and Specht (2010, Solarzeitalter, 1(10):51-58) describe a method for integration and storage of renewable energies and a route to full supply based on renewable energies. They concentrate on the increase of fluctuations in the German power supply and suggest storage of energy of renewable energy sources via methane which is produced by chemical synthesis.

US 2009/0130734 A1 describes a method of converting carbon dioxide gas produced during industrial processes comprising contacting methanogenic archaea with the carbon dioxide gas under suitable conditions to produce methane.

Although different approaches exist which aim at the utilization of renewable energy sources, these methods - nearly without exception - suffer from low energy recovery rates on the one hand and high production cost on the other hand. Therefore, these applications are far away from being economic. And as long as these methods and systems are not economic, they are not widely applicable or suitable for world-wide energy supply and of course not competitive to fossil fuels or nuclear power. Hence, there is still a high need for a system and method which provide energy from a renewable or non-renewable energy source in a storable and transportable form that is also inexpensive, has high energy conversion rates and is environmentally friendly, i.e. at least neutral in carbon dioxide release and low in the release of other environmentally harmful or polluting substances.

Thus, optimal connection and exploitation of educts, intermediates and products are key features of the method of the invention for economically using renewable energy sources in order to replace fossil fuels and also nuclear energy as main energy sources. Only this guarantees an optimal use of natural resources and thus of energy. None of the components remains unused or is considered as waste. Moreover, especially substances which are considered waste in other industries are valuable educt components for the present invention and therefore educts for the method of the invention. First energy of a renewable or non-renewable energy source is used, preferably of a renewable energy source. However, it may also be beneficial to convert energy of a non-renewable source into methane in periods of low energy consumption or low net utilisation. Second the energy of the renewable or non-renewable energy source is used for the electrolysis of water or brine to produce hydrogen on the one hand and oxygen (in case of the electrolysis of water) on the other hand. The hydrogen obtained by the electrolysis is then used for the conversion of hydrogen and carbon dioxide into methane which is an ideal energy carrier for which a well-developed infrastructure, storage facilities and end-user applications already exist. Thus, the method and system presented herein are already applicable in common transportation and heating systems, whereas other approaches aiming at solving the problem of energy supply in the future, require the development of new engines or heating system in the first place and are therefore not applicable immediately.

The present invention provides a method system for converting energy from any energy source, such as renewable or non-renewable energy sources, in particular of a renewable energy source, in an inexpensive way and with high process efficiency in a storable and transportable form and thus solves the problem of energy storage, allows for transportation of such energy carrier in current infrastructure and easy delivery to the end-user. Also disclosed is a respective system. For this purpose, the energy from a renewable energy source is converted into methane as end-product. Methane is storable and easy to transport with a well-developed infrastructure which is already available. It is an important fuel and energy source and the major component of natural gas. Methane burns with oxygen and solely produces water and carbon dioxide ($CO_2$). It is the major component of natural gas, wherein the methane content varies between 70% and 99%. Natural gas exhibits the cleanest burning properties when compared to other fossil fuels like oil or coal and produces less $CO_2$ per energy unit released.

Methane is effectively produced from $CO_2$ either chemically by the so-called Sabatier reaction or Sabatier process or biologically by methanogenic microorganisms which are capable of methanogenesis. Methanogenesis is a biological pathway that converts $CO_2$ and hydrogen ($H_2$) into methane ($CH_4$) according to the overall reaction $CO_2 + 4H_2 \rightarrow CH_4 + 2H_2O$. Using methanogenic microorganisms instead of the chemical Sabatier process, the reaction takes place at relatively moderate temperatures. The reaction system comprising the methanogenic microorganisms is robust with a good impurity tolerance and has a high selectivity even at normal pressure. For this reasons, the method of the present invention employs the biological production of methane rather than the chemical.

For the efficient methane production by methanogenic microorganisms by methanogenesis, the invention not merely refers to methods in the art but provides an optimized methane production method which may preferably, but optionally be introduced in the overall method of the invention for storing energy in the form of methane and advantageously adds to the overall efficiency of the method, thereby increasing the method's outcome, its profitability and enhances its environment protective effect. The inventor has surprisingly found out that a specific ratio of the partial pressure of $H_2$ to the partial pressure of $CO_2$ is the most appropriate way to control the actual fermentation condition and gas ratio inside the reaction vessel and thus the provision of educts to the methanogenic microorganisms. The "partial pressure" of an ideal gas in a gas mixture is equal to the pressure it would exert if the same volume is occupied alone at the same temperature. This is because ideal gas molecules are so far apart that they do not interfere with each other. Real gases come very close to this ideal and the gas phase above the liquid phase inside the reaction vessel has to be regarded as real gas phase in a thermodynamic definition. In the prior art, if any, simply the volumetric inflow ratio of both gases has been considered, however, not the real gas supply situation inside the reaction vessel.

Thus, no information on the actual gas ratio inside the reaction vessel is provided in the literature. The actual gas ratio inside of the reaction vessel is more important than the simple gas feed ratio since it reflects the real situation as to how the methanogenic microorganism operates. The partial pressures are linked via Henry's law to the solubility of the gaseous components in the liquid phase, the concentrations the cell actually experiences during the biological reaction. The partial pressure of a gas dissolved in a liquid is the partial pressure of that gas which would be generated in a gas phase in equilibrium with the liquid at the same temperature. Gases dissolve, diffuse, and react according to their partial pressures, and not necessarily according to their concentrations in a gas mixture. By controlling partial pressure ratio rather than volumetric amounts, the part of methane production by conversion of hydrogen and carbon dioxide can significantly be improved.

However, to connect the production of methane by methanogenic microorganisms with a renewable or non-renewable energy source, at least one intermediate step is necessary: The use of the electric energy which is obtained from the energy source for the electrolysis of water or brine to obtain hydrogen in the first place and optionally also oxygen. Together with carbon dioxide, at least this hydrogen is used for the production of methane by methanogenic microorganism, subsequently. Also the oxygen which is provided by the electrolysis of water or brine may optionally be introduced in the method of the invention for optimal use of all reaction components. In oxygen enriched combustion, this oxygen is used for producing carbon dioxide as will be explained in detail below.

Third the carbon dioxide which is used for the methanogenesis may at least partially be derived from any source, for example from an industrial source or an industrial process where carbon dioxide is - for example - considered as waste or by-product which is released to the atmosphere, or the carbon dioxide may be produced in oxygen enriched combustion, wherein at least the oxygen obtained by electrolysis of water is used for producing carbon dioxide. Otherwise a release of carbon dioxide would lead to a net increase of the amount of carbon dioxide and thus of greenhouse gases in the atmosphere which probably contributes to global warming. This effect may at least be partially prevented by applying the method of the invention which re-uses such "waste" carbon dioxide.

To overcome the above mentioned drawbacks in current energy utilisation, recycling of waste material and to improve the protection of the environment, the invention provides a method for storing energy in the form of methane comprising at least the following steps:

a) generating electric energy using a renewable and/or non-renewable energy source, preferably generating electric energy using a renewable energy source;
b) using at least partially the electric energy of step a) for the electrolysis of water and/or brine, thereby producing hydrogen and/or oxygen; and
c) using at least or at least partially the hydrogen of step b) and carbon dioxide, and optionally partially the electric energy of step a), for producing methane by methanogenic microorganisms in a reaction vessel, comprising contacting the methanogenic microorganisms with an in-gas feed comprising at least said hydrogen and carbon dioxide, optionally step c) further comprising at least one methane production phase and/or at least one cell growth phase.

In a preferred embodiment, the method further comprises step d), wherein at least the oxygen of step b) is used for producing at least partially the carbon dioxide used in step c) by oxygen enriched combustion or gasification. Thus, the carbon dioxide which is used in step c) for producing methane by methanogenic microorganisms in a reaction vessel is at least partially obtained by oxygen enriched combustion or gasification. Alternatively or in addition, the carbon dioxide used in method step c) for producing methane by methanogenic microorganisms in a reaction vessel is at least partially from or partially derived from an industrial process, is off-gas of step c) of the invention, real gas or a mixture thereof. Optionally, the industrial process comprises biogas or biomass fermentation, anaerobic digestion, the electrolysis of water or brine, methanogenesis by methanogenic archaea, oxygen enriched combustion gasification, and/or combinations thereof. Optionally, the industrial process is selected from the group consisting of biogas or biomass fermentation, anaerobic digestion, the electrolysis of water or brine, methanogenesis by methanogenic microorganisms, such as methanogenic archaea, oxygen enriched combustion, gasification, and/or combinations thereof. Preferably, the electric energy of step a) and/or for the industrial process is generated using a renewable energy source or renewable energy device.

In any of the above embodiments of the method of the invention, the renewable energy source comprises solar energy, wind power, wave power, tidal power, water or hydro power, geothermal energy, biomass and biofuel combustion, or combinations thereof.

In any of the above mentioned embodiments of the method of the invention, the hydrogen used in step c) for producing methane by methanogenic microorganisms in a reaction vessel is at least or at least partially produced by the electrolysis of water or brine, preferably in step b) of the invention. Thus, the hydrogen which is used in step c) for producing methane by methanogenic microorganisms in a reaction vessel is at least partially obtained by electrolysis of water and/or brine. Alternatively or in addition, the hydrogen used in method step c) for producing methane by methanogenic microorganisms in a reaction vessel is at least from and/or is derived from an industrial process, is off-gas, real gas or a mixture thereof. Optionally, the industrial process comprises biogas or biomass fermentation, anaerobic digestion, the electrolysis of water or brine, methanogenesis by methanogenic archaea, oxygen enriched combustion or gasification, and/or combinations thereof. Optionally, the industrial process is selected from the group consisting of biogas or biomass fermentation, anaerobic digestion, the electrolysis of water or brine, methanogenesis by methanogenic archaea, oxygen enriched combustion or gasification, and/or combinations thereof. In another preferred embodiment of the method of the invention, the energy for the industrial process or the electrolysis of water or brine to obtain hydrogen and/or oxygen is derived from a renewable energy source, such as solar energy, wind power, wave power, tidal power, water or hydro power, geothermal energy, biomass and biofuel combustion, or combinations thereof.

Further, in any embodiment of the method of the invention, the methanogenic microorganisms are methanogenic archaea, preferably the methanogenic archaea is selected from the group consisting of *Methanosarcinia barkeri*, *Methanobacterium thermoautotrophicus*, *Methanothermobacter thermoautotrophicus*, *Methanococcus maripaludis*, *Methanothermobacter marburgensis, Methanocaldococcus jannaschii,* and mixtures thereof, even more preferably, the methanogenic archaea is *Methanothermobacter marburgensis,* most preferably, the methanogenic archaea is *Methanothermobacter marburgensis* strain DSM 2133.

According to the invention, step c) for producing methane by methanogenic microorganisms in a reaction vessel comprises at least one methane production phase, wherein in the at least one methane production phase the ratio of the partial pressure of hydrogen to the partial pressure of carbon dioxide inside the reaction vessel is maintained at 5:1 or higher (parts hydrogen : parts carbon dioxide), preferably the ratio of the partial pressure of hydrogen to the partial pressure of carbon dioxide is maintained in the range from 7:1 to 30:1 (parts hydrogen : parts carbon dioxide), more preferably in the range from 9:1 to 25:1 (parts hydrogen : parts carbon dioxide), even more preferably in the range from 10:1 to 18:1 (parts hydrogen : parts carbon dioxide), most preferably, the ratio of the partial pressure of hydrogen to the partial pressure of carbon dioxide inside the reaction vessel in the at least one methane production phase is maintained in the range from 12:1 to 17:1 or adjusted to 15±1:1 or 18±1:1 (parts hydrogen : parts carbon dioxide).

In any of the above mentioned embodiments of the method of the invention, optionally in step c) for producing methane by methanogenic microorganisms in a reaction vessel in the at least one methane production phase, the oxidation reduction potential inside the reaction vessel is maintained below -350 mV, preferably the oxidation reduction potential is maintained in the range from -400 mV to -700 mV, more preferably, the oxidation reduction potential inside the reaction vessel is maintained in the range from -500 mV to -700 mV, most preferably, maintained at a value of -550±50 mV.

In an optional embodiment of the method of the invention, step c) for producing methane by methanogenic microorganisms in a reaction vessel further comprises at least one cell growth phase, wherein in the at least one cell growth phase the ratio of the partial pressure of hydrogen to the partial pressure of carbon dioxide inside the reaction vessel is maintained at a ratio different from the ratio in the at least one methane production phase, preferably, the ratio of the partial pressure of hydrogen to the partial pressure of carbon dioxide is lower than in the at least one methane production phase, more preferably, the ratio of the partial pressure of hydrogen to the partial pressure of carbon dioxide is maintained at 4:1 or lower (parts hydrogen : parts carbon dioxide), even more preferably, in the at least one cell growth phase the ratio of the partial pressure of hydrogen to the partial pressure of carbon dioxide is maintained in the range from 0.5:1 to 4:1 or from 0.75:1 to 3.5:1 (parts hydrogen : parts carbon dioxide), also even more preferably, maintained in the range from 1:1 to 2.5:1 (parts hydrogen : parts carbon dioxide), most preferably, the ratio of the partial pressure of hydrogen to the partial pressure of carbon dioxide inside the reaction vessel in the at least one cell growth phase is maintained at 1.5±0.5:1 or 2±1:1 (parts hydrogen : parts carbon dioxide). Further ratios for hydrogen and carbon dioxide for both phases, the methane production phase and the cell growth phase, are given below.

In any of the above mentioned embodiments of the method of the invention, wherein step c) for producing methane by methanogenic microorganisms in a reaction vessel further comprises at least one cell growth phase, optionally, the oxidation reduction potential in the at least one cell growth phase is maintained below -300 mV, preferably, the oxidation reduction potential in the at least one cell growth phase is maintained in the range from -300 mV to -500 mV, even more preferably, the oxidation reduction potential inside the reaction vessel is maintained in the range from -300 mV to -400 mV, most preferably, the oxidation reduction potential in the at least one cell growth phase inside the reaction vessel is maintained at a value of -350±25 mV.

In any above mentioned embodiment, the oxidation reduction potential is adjusted using a reducing agent, such as sodium sulphide or hydrogen sulphide, or by adjusting the hydrogen or oxygen in-gas feed into the reaction vessel.

In one exemplary embodiment, step c) of the method of the invention comprises at least one cell growth phase and at least one methane production phase, preferably, step c) comprises at least one cell growth phase, wherein the ratio of the partial pressure of hydrogen to the partial pressure of carbon dioxide is maintained at 4:1 or lower (parts hydrogen : parts carbon dioxide), and at least one methane production phase, wherein the ratio of the partial pressure of hydrogen to the partial pressure of carbon dioxide is maintained at 5:1 or higher (parts hydrogen : parts carbon dioxide).

In another optional embodiment, step c) of the method of the invention comprises or consists of the following steps in the order given

a) at least one cell growth phase, wherein the ratio of the partial pressure of hydrogen to the partial pressure of carbon dioxide is maintained at a ratio of 4:1 or lower (parts hydrogen : parts carbon dioxide), and

b) at least one methane production phase, wherein the ratio of the partial pressure of hydrogen to the partial pressure of carbon dioxide is maintained at 5:1 or higher (parts hydrogen : parts carbon dioxide).

In any of the above mentioned embodiments of the method of the invention, the pH value inside the reaction vessel in step c) of the method is maintained in the range from 5.0 to 8.0, preferably in the range from 5.5 to 7.8, more preferably in the range from 6.4 to 7.6, even more preferably in the range from 6.7 to 7.2, and most preferably the pH inside the reaction vessel is maintained at 6.9 ± 0.5.

In any embodiment of the method of the invention, wherein step c) comprises at least one cell growth phase, the methanogenic microorganisms are maintained in the at least one cell growth phase until the cell growth of the methanogenic microorganisms in the reaction vessel stagnates or reaches at least a concentration of 3 g biomass per litre.

In another preferred embodiment of the method of the invention, in step c) of the method the methanogenic microorganisms are switched from at least one cell growth phase to at least one methane production phase by adjusting or maintaining the ratio of the partial pressure of hydrogen to the partial pressure of carbon dioxide inside the reaction vessel to or at 5:1 or higher (parts hydrogen : parts carbon dioxide) or from at least one methane production phase to at least one cell growth phase by adjusting or maintaining the ratio of the partial pressure of hydrogen to the partial pressure of carbon dioxide inside the reaction vessel to or at a ratio different from the ratio in the at least one methane production phase, optionally, in step c) of the method for switching the methanogenic microorganisms from at least one cell growth phase to at least one methane production phase the oxidation reduction potential is adjusted to or maintained at a value below -350 mV and/or for switching the methanogenic microorganisms from at least one methane production phase to at least one cell growth phase the oxidation reduction potential is adjusted to or maintained at a value below - 300 mV, optionally, using a reducing agent, such as sodium sulphide or hydrogen sulphide, by adjusting the oxygen in-gas feed, the hydrogen in-gas feed and/or pH value as will be explained in detail below.

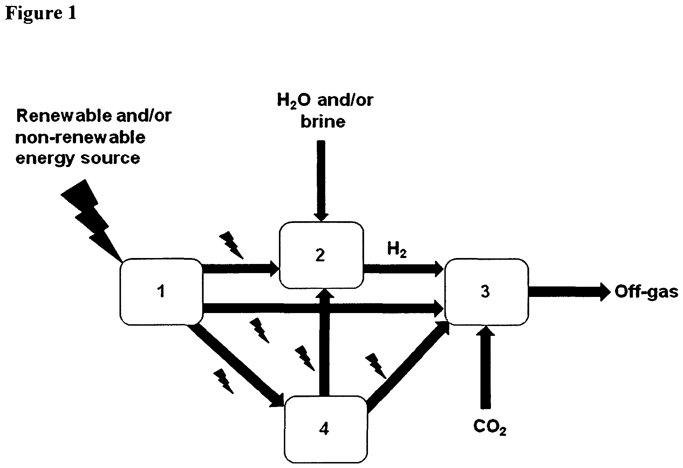

Further disclosed is a system for storing energy in the form of methane comprising at least one device for generating electric energy from a renewable and/or non-renewable energy source, at least one device for producing hydrogen and/or oxygen by the electrolysis of water or brine, and at least one bioreactor comprising a reaction vessel suitable for growing, fermenting and/or culturing methanogenic microorganisms, optionally for converting hydrogen and carbon dioxide into methane. This system allows for performing and/or conducting the method of the invention. A general set-up of the system is illustrated in Figure 1 and comprises at least one device for generating electric energy from a renewable and/or non-renewable energy source (1), at least one device for producing hydrogen and/or oxygen by the electrolysis of water and/or brine (2), preferably said device for producing hydrogen and/or oxygen by the electrolysis of water and/or brine is an electrolyser, and at least one bioreactor (3) comprising a reaction vessel suitable for growing, fermenting and/or culturing methanogenic microorganisms for converting hydrogen and carbon dioxide into methane (Figure 1). The bioreactor further comprises at least one device for measuring the head pressure and the off-gas concentration and at least one device for adjusting or maintaining the partial pressure ratio of hydrogen to carbon dioxide inside the reaction vessel. It should be noted that more than one device for generating electric energy from a renewable and/or non-renewable energy source (1), more than one device for producing hydrogen and/or oxygen by the electrolysis of water and/or brine (2), preferably electrolyser, and/or more than one bioreactor (3) comprising one or more reaction vessel suitable for growing, fermenting and/or culturing methanogenic microorganisms for converting hydrogen and carbon dioxide into methane may be present in the system. "More than one" denotes two, three, four, five, and so on. Of course, the particular number of the devices for a certain purpose, i.e. for generating electric energy, for producing hydrogen and/or oxygen or the bioreactor may be selected individually. Further, in case that more than one device for generating electric energy from a renewable and/or non-renewable energy source is used in the system of the invention, of course, devices for generating electric energy from different renewable and/or non-renewable energy sources may be used. In case that more than one bioreactor is present in the system it is also applicable that one or more bioreactor is used for the cell growth phase and one or more other bioreactor is used for the methane production phase. Even different ratios of the partial pressure of hydrogen to carbon dioxide may be applied in the reaction vessels of bioreactors which are used for the same phase of the invention. In case of the electrolysis of water, wherein oxygen and hydrogen are produced, both hydrogen and oxygen are separated by means known to a person skilled in the art and discussed in detail below as only the hydrogen should be introduced into the bioreactor, whereas the oxygen is optionally used in gasification or oxygen enriched combustion processed as also discussed in detail below. Optionally the renewable energy source comprises solar energy, wind power, wave power, tidal power, water/hydro power, geothermal energy, biomass and biofuel combustion. Arrows indicate the transfer of one or more compounds (e.g. electric energy, liquid, gaseous and/or solid compounds) between the devices/bioreactor(s) or else of the system. The electric energy from a renewable and/or non-renewable energy source, may be transferred via general power supply lines or cables or else to the at least one device for producing hydrogen and/or oxygen by the electrolysis of water and/or brine (2), preferably said device for producing hydrogen and/or oxygen by the electrolysis of water and/or brine is an electrolyser, and/or to the at least one bioreactor (3) or be stored in an electric energy storage device (4), such as a battery or accumulator or else. In general and applicable to all the electric energy storage device (4), such as a battery or accumulator or else may be any common device suitable for storing electric energy, such as a common battery or common accumulator to be used according to the manufacturer's instructions. From the electric energy storage device (4) the electric energy may be transferred via general power supply lines or cables or else to the at least one device for producing hydrogen and/or oxygen by the electrolysis of water and/or brine (2), preferably said device for producing hydrogen and/or oxygen by the electrolysis of water and/or brine being an electrolyser, and/or to the at least one bioreactor (3).

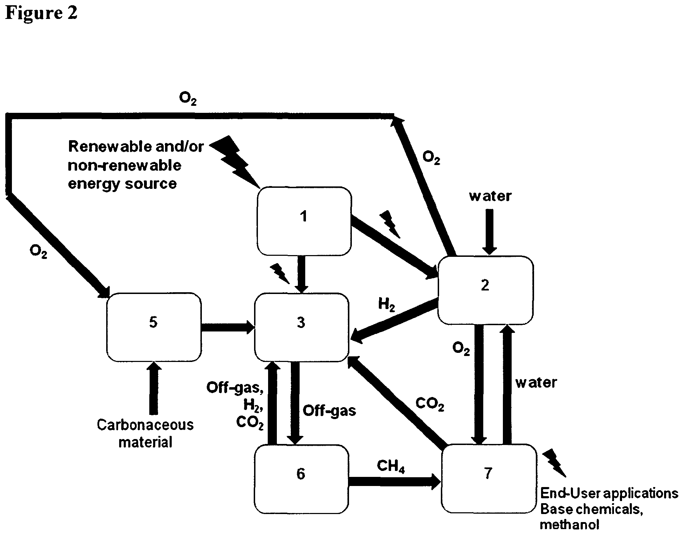

Water and/or brine are provided via general transportation or tubes, pipes or else but also via water tanks transported by transportation systems such as trains, cars, trucks or else. Water in general, and in particular water from rain or water which was purified in order to be suitable for the use in electrolysis may be stored in water tanks, cisterns, artificial lakes, reservoirs or else. The hydrogen obtained by electrolysis is transferred to the bioreactor (3), where it is converted together with carbon dioxide into methane by methanogenic microorganisms. Hydrogen, carbon dioxide and also other gaseous substances, such as oxygen, may in general be transferred via standard gas transport means, such as tubes, pipes or else. They may also be delivered, for example, in gas cylinders or bottles which allow for storage of carbon dioxide and/or hydrogen before introduction into the bioreactor. Gas cylinders and/or bottles allow not also for storage of carbon dioxide prior to introduction into the bioreactor, such as for example of hydrogen after electrolysis in case of excess production or else, but also for individual transportation of carbon dioxide and/or hydrogen to bioreactors (3) which are - for example - not connected to any gas transport means, such as tubes, pipes or else. The carbon dioxide may be of any source as will be further discussed in detail below, optionally the carbon dioxide used herein is air or carbon dioxide waste gas from an industrial process. The off-gas is removed from the bioreactor (3) via the standard means of the bioreactor (3), such as tubes, pipes or else. These standard means are familiar to a person skilled in biotechnology and can also be taken from the bioreactor manufacturer's instructions. The off-gas comprises methane, water vapour, hydrogen and carbon dioxide but also other gases which are present inside the reaction vessel as described below. The off-gas may be further purified or enriched (by at least partially removing components except methane) for increasing the methane content by methods well known to a person skilled in the art and further discussed in detail below. The off-gas or the methane-enriched off-gas may also be stored in gas cylinders, bottles or else or it may be recycling and/or further processed in order to also use gaseous components other than methane, such as carbon dioxide and/or hydrogen which have not been converted into methane by the methanogenic microorganisms. The off-gas may also be led back into the bioreactor by standard means and devices or it is transferred into another bioreactor. Or the off-gas, methane or methane-enriched off-gas may be used directly and/or after transportation in gas cylinders, bottles or else or by standard gas transport means, such as tubes, pipes or else, as fuel or energy carrier in end-user applications, for heating devices, vehicles, or be converted into other forms of energy, such as thermal, mechanical, electric energy or else. Methane burns with oxygen, such as comprised in air or produced by electrolysis of water, to carbon dioxide, which may be re-used for methane production in the bioreactor by the methanogenic microorganisms, and water, which may be re-used for electrolysis for hydrogen and oxygen production. The overall process and an exemplary set-up of the system are illustrated in Figure 2. Thereby, none of the substances or components is wasted. Another end-user application of methane is the conversion into methanol via a three step reaction as follows: (i) Steam reforming: $CH_4 + H_2O \leftrightarrow CO + 3H_2$ (requires Ni catalyst and 800 °C), (ii) water shift reaction: $CO + H_2O \leftrightarrow CO_2 + H_2$ (requires Ni catalyst and 800 °C) and (iii) synthesis: $2H_2 + CO \leftrightarrow CH_3OH$. The methanol may further be converted to gasoline by the Mobil process. Methanol may also be used as educt for the production of base chemicals (e.g. olefin via "methanol to olefin process" and access to solid polymers like polypropylene). All these methods are known to a person skilled in the art. Information on the so-called "methanol to olefin process" may for example be obtained from UOP Corporate Offices, Des Plaines, IL, USA) or from Norsk Hydro ASA, Oslo, Norway.

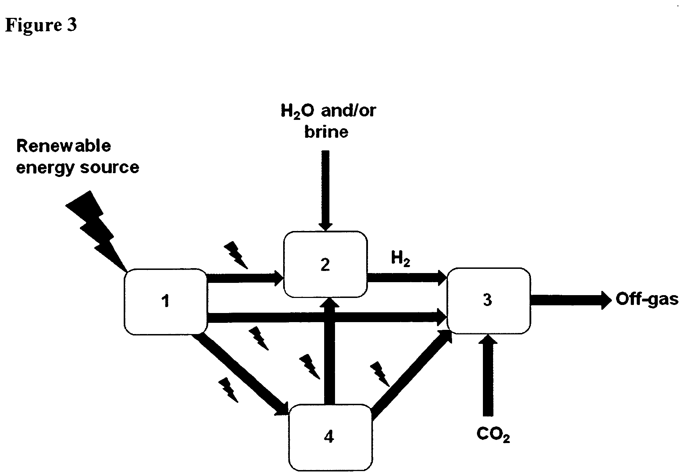

The system for storing energy in the form of methane comprises at least one device for generating electric energy from a renewable energy source, optionally the renewable energy source comprises solar energy, wind power, wave power, tidal power, water/hydro power, geothermal energy, biomass and biofuel combustion, at least one device for producing hydrogen and/or oxygen by the electrolysis of water and/or brine, and at least one bioreactor comprising a reaction vessel suitable for growing, fermenting and/or culturing methanogenic microorganisms for converting hydrogen and carbon dioxide into methane. The general set-up of this preferred variation of the system of the invention is illustrated in Figure 3. All explanations regarding the general set-up of the system as in Figure 1 also apply with respect to the exemplary system as in Figures 2, 3 and 4.

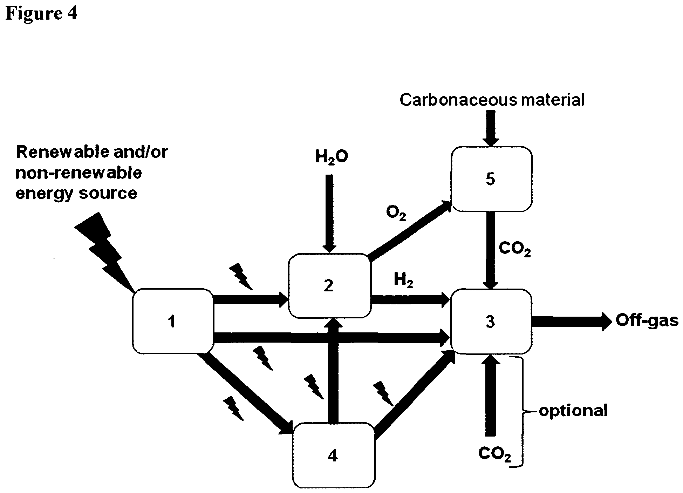

The system may further comprise at least one device for oxygen enriched combustion or gasification (5) for producing carbon dioxide, preferably, the device for oxygen enriched combustion or gasification (5) uses the oxygen produced by the electrolysis of water in device (2) for producing carbon dioxide, said carbon dioxide being transferred to the bioreactor (3), thereby being transferred to the methanogenic microorganisms inside the reaction vessel comprised in the bioreactor (3). An exemplary scheme is comprising at least one device for oxygen enriched combustion (5) or gasification (5) for producing carbon dioxide is illustrated in Figure 4. All explanations regarding the general set-up of the system as in Figure 1, 2 or 3 also apply with respect to the exemplary system as in Figure 4. As already described above, hydrogen, carbon dioxide and also other gaseous substances, such as oxygen, may in general be transferred via standard gas transport means, such as tubes, pipes or else. They may also be delivered, for example, in gas cylinders or bottles which allow for storage of the substance before introduction into the bioreactor in case of hydrogen and carbon dioxide or delivered to combustion or gasification in case of the oxygen derived from the electrolysis of water. Gas cylinders and/or bottles not only allow for the storage of carbon dioxide prior to introduction into the bioreactor (3), such as for example of hydrogen after electrolysis in case of excess production or else, but also for the individual transportation of carbon dioxide and/or hydrogen to bioreactors (3) which are - for example - not connected to any gas transport means, such as tubes, pipes or else. Optionally, additional carbon dioxide from any other carbon dioxide source mentioned herein may be introduced into the bioreactor (3) and thereby to the methanogenic microorganisms comprised in the reaction vessel comprised in the bioreactor. This may be applicable in case of low carbon dioxide production by the combustion or gasification device (5). For combustion/oxygen enriched combustion or gasification, carbonaceous material, e.g. petroleum, wood, coal, living and/or dead biomass, is required and may be delivered via standard transportation as known to a person skilled in the art.

The system may further comprise one or more devices for purifying carbon dioxide of any source and also mixtures thereof from contaminants prior to its feed into the reaction vessel of the bioreactor of the invention. For example, such a carbon dioxide purifying device may be present between the device for oxygen enriched combustion or gasification and the bioreactor. Such devices and methods are known to a person skilled in the art, such as a carbon dioxide scrubber, contacting the gas with an absorbing medium which selectively absorbs carbon dioxide, such as amine, monoethanolamine solutions or quicklime absorption, or which selectively absorbs gases other than carbon dioxide from the real gas, activated charcoal or activated coal, by the regenerative carbon dioxide removal system, polymer membrane gas separators, molecular sieves, others and combinations thereof.

In addition, the system may further comprise at least one device for recovery, purification, enriching, storing, recycling and/or further processing of off-gas, hydrogen, water, oxygen, chlorine, electric energy, carbon dioxide, $H_2S$, sodium sulphite, methanogenic microorganisms, used medium and medium components, methane and other substances of the process. These devices for recovery, purification, enriching, storing, recycling and/or further processing are connected to the respective device or to the bioreactor where the above listed substance occurs or can easily obtained. They may be removed from the system of the invention using devices and/or means which are suitable for this purpose depending on the nature of the particular substance, such as cables or lines for electric energy or pipes, tubes and else for liquid and/or gaseous substances. "Substances of the process" or "compounds of the process" denote all liquid, gaseous and/or solid substances and also solutions, cells, medium, medium components and suspensions comprising methanogenic microorganisms of the present invention which are introduced into the system and/or produced by the methanogenic microorganisms of the invention or by any other chemical or biological reaction which may occur inside the system of the invention, in particular inside the reaction vessel and/or the device for electrolysis of water or brine. Substances of the process are also substances which occur only as intermediates of the method. Hydrogen, water, oxygen, chlorine, carbon dioxide, $H_2S$, off-gas, sodium sulphite, methanogenic microorganisms, used medium and medium components, methane, electric energy and others are exemplary substances of the process. It is of particular importance in the method and also the system of the invention that all educts, products and intermediates are optimally used. Therefore, especially the off-gas is further processed, methane purified and other components recycled. How especially carbon dioxide, oxygen and hydrogen are recovered from the off-gas and re-used in the method of the invention is described in detail below. In the system, the methanogenic microorganisms are methanogenic archaea, preferably the methanogenic archaea is selected from the group consisting of *Methanosarcinia barkeri, Methanobacterium thermoautotrophicus*, *Methanothermobacter thermoautotrophicus*, *Methanococcus maripaludis*, *Methanothermobacter marburgensis*, *Methanocaldococcus jannaschii*, and mixtures thereof, even more preferably, the methanogenic archaea is *Methanothermobacter marburgensis*, most preferably, the methanogenic archaea is *Methanothermobacter marburgensis* strain DSM 2133.

A bioreactor which is suitable for step c) of the method of the invention and may optionally be comprised in the system of the invention is described. The bioreactor comprises at least

a) at least one reaction vessel for fermenting, growing and/or culturing methanogenic microorganisms,
b) at least one device for introducing and removing gaseous substances into the at least one reaction vessel,
c) at least one device for introducing or removing a liquid substance or a cell suspension into or from the bioreactor or reaction vessel,
d) devices for measuring and/or adjusting at least the oxidation reduction potential, temperature, pressure, head pressure, off-gas concentration and content, the partial pressure ratio of hydrogen and carbon dioxide and/or pH value,
e) at least one device for removing off-gas from the at least one reaction vessel,
f) at least one stirring device inside the at least one reaction vessel, and/or
g) at least one device for gas liquid mass transfer.

A culture of methanogenic microorganisms having a biomass concentration of at least 6 g biomass per litre is described, wherein no additional methanogenic microorganism is added after inoculation or cell retention is applied during step c) of the invention, and uses thereof for converting hydrogen and carbon dioxide into methane.

The invention provides a method comprising a step c) of producing methane by converting hydrogen and carbon dioxide to methane by methanogenic microorganisms in a reaction vessel. Step c) of the method of the invention comprises contacting the methanogenic microorganisms in the cell culture with a gas feed comprising hydrogen and carbon dioxide and comprises at least one methane production phase and optionally at least one cell growth phase. In the at least one methane production phase of the invention, the partial pressure ratio of hydrogen to carbon dioxide ($H_2/CO_2$ ratio) in the reaction vessel is maintained at 5:1 or higher (parts hydrogen : parts carbon dioxide), preferably, maintained in the range from 7:1 to 30:1 (parts hydrogen : parts carbon dioxide), more preferably in the range from 9:1 to 25:1 (parts hydrogen : parts carbon dioxide), even more preferably in the range from 10:1 to 18:1 (parts hydrogen : parts carbon dioxide), most preferably, the ratio of the partial pressure of hydrogen to the partial pressure of carbon dioxide inside the reaction vessel is maintained in the range from 12:1 to 17:1 or adjusted to about $15\pm1{:}1$ or $18\pm1{:}1$ (parts hydrogen : parts carbon dioxide) in the at least one methane production phase, whereas in the optional at least one cell growth phase, the partial pressure ratio of hydrogen to carbon dioxide is different from the ratio in the at least one methane production phase, preferably, the partial pressure ratio of hydrogen to carbon dioxide is maintained at or adjusted to a ratio lower than in the ratio in the at least one methane production phase, particularly preferably, the partial pressure ratio is maintained at or adjusted to 4:1 or lower (parts hydrogen : parts carbon dioxide), more preferably, the partial pressure ratio is maintained at or adjusted to a ratio in the range from 0.5:1 to 4:1 or from 0.75:1 to 3.5:1 (parts hydrogen : parts carbon dioxide), even more preferably, the partial pressure ratio is maintained at or adjusted to a ratio in the range from 1:1 to 2.5:1 (parts hydrogen : parts carbon dioxide), most preferably, the partial pressure ratio is maintained at or adjusted to a ratio of $1.5\pm0.5{:}1$ or $2\pm1{:}1$ (parts hydrogen : parts carbon dioxide) in the at least one cell growth phase.

In this way, methane is produced which is the major component of natural gas and a valuable energy carrier. Moreover, the method of the invention consumes carbon dioxide and thus reduces the amount of greenhouse gases in the atmosphere. The method of the invention, in particular in step c), uses carbon dioxide and hydrogen from any source and thus provides a method for reducing the amount of carbon dioxide released and hence the amount of carbon dioxide in the atmosphere which is suspected by many researches to cause global warming. Another aspect of the invention is the conversion of carbon dioxide contained in waste gas from any industrial process, such as coal gasification, biomass gasification, liquid fuel production by biomass fermentation, ethanol production from biomass and others into methane. Thus, the invention has a double positive effect on the environment as it uses renewable energy in a preferred embodiment and also helps to reduce the amounts of waste and the release of greenhouse gases or other substances in the atmosphere.

The inventor has surprisingly found out that the key parameter for an economically feasible methane production is the volumetric productivity. The volumetric productivity is calculated from equation (1):

$$\text{Volumetric productivity [mmol/L/h]}$$
$$=$$
$$(\text{biomass concentration [g/L]}) \times (\text{specific methane production rate [mmol/g/h]}). \quad (1)$$

As can be learned from the equation developed by the inventor, the volumetric productivity depends on the biomass concentration of the methanogenic microorganisms in the reaction vessel of the invention and on the specific methane production rate. The "specific methane production rate" denotes the amount of methane [mmol] produced per gram of methanogenic microorganisms, i.e. biomass [g] per hour [h]. Thus, on the one hand, the cell concentration should be as high as possible in the method and in the reaction vessel of the invention due to the fact that the cells, i.e. the biomass of methanogenic microorganisms, principally function as a catalyst for converting hydrogen and carbon dioxide into methane. On the other hand, the specific methane production rate reflects the productivity per gram biomass of methanogenic microorganism, i.e. the catalytic productivity of the biomass inside the reaction vessel.

The inventor has further surprisingly found out that both parameters, the biomass concentration and the specific methane production rate, and therefore also the volumetric productivity, depend on the ratio of the partial pressures of hydrogen and carbon dioxide inside the reaction vessel. Thus, the invention described herein allows a very specific control of the fermentation process and methane production, wherein the experimenter can specifically control the carbon flux from carbon dioxide either to biomass production in a cell growth phase or to methane production in a methane production phase. To date, the ratio of the partial pressure of hydrogen and carbon dioxide has never been regarded as having an impact on methane production by methanogenic microorganisms.

The partial pressure of a gas reflects the specific, i.e. partial, pressure of said gas in a gas mixture, here in the gas phase inside the reaction vessel. In the reaction vessel, essentially a liquid phase and a gaseous phase are present. The gaseous phase comprises the gases which are fed into the reactor or are produced by the methanogenic microorganisms. The liquid phase inside the reaction vessel comprises the methanogenic microorganisms of the invention which are typically suspended in a standard medium. The partial pressures of the gases are determined by a combination of sensors which measure the head pressure and the off-gas concentration, and thus may be adjusted in real time to a specific ratio or maintained at a value or in a specific range. "Head pressure" denotes the total pressure inside the reaction vessel. Much better than simply the adjustment of the volumetric ratio of the gas inflow, the measurement and adjusting or maintaining of the ratio of the partial pressures of at least carbon dioxide and hydrogen allows for a specific transport of the gases to the methanogenic microorganisms inside the reaction vessel. Thereby, different sources of carbon dioxide and hydrogen with different content of said gases may easily be used, mixed and exchanged as the ratio of gas which is provided to the methanogenic microorganisms may simply be adjusted or maintained according to real measurement. This invention presents for the first time a method for converting hydrogen and carbon dioxide into methane by methanogenic microorganisms, wherein it is not even necessary to determine the exact composition of the gas source which is used, e.g. biogas. Due to the novel and inventive approach of the method, wherein the pressure ratio of both gases in inside the reaction vessel is adjusted to or maintained at a specific value or in a range according to the desired carbon flux, i.e. towards cell growth in the cell growth phase or towards methane production in the methane production phase.

The partial pressures of both gases, hydrogen and carbon dioxide, which are the methanogenesis educts for the conversion to methane by the methanogenic microorganisms in step c) of the method of the invention, provide much more reliable information on how much of the two educt substances is actually provided to the cells. At a known temperature, the partial pressures of the gases also allow the calculation of the dissolved gas concentrations which are actually available to the cells since partial pressures are linked via Henry's law to the solubility of the gaseous components in the liquid phase. The partial pressures of the gases are determined by a combination of sensors which measure the head pressure and the off-gas concentration, and thus may be adjusted or maintained in real time to a specific ratio or in a specific ratio range. For the measurement of the off-gas concentrations standard gas analyzer systems (such as provided by BlueSens gas sensor GmbH, Herten, Germany) can be used. For measurement of the head pressure standard pressure sensors such as provided by Keller (Winterthur, Switzerland) are used either directly inside the reaction vessel or for off-gas measurement inside the device for removing the off-gas from the reaction vessel. The partial pressures are derived in real time in a process management system (Lucullus; Biospectra AG, Schlieren, Switzerland) according to the equation $p_i = c_i/p_{total}/100$, wherein i specifies the respective component, $c_i$ is the gas concentration of component i in volume % and $p_{total}$ is the total head pressure in bar absolute.

Surprisingly, the inventor found out that the methanogenic microorganisms require a different partial pressure of carbon dioxide and hydrogen for optimal cell growth, i.e. biomass concentration increase, than for optimal methane production. A determined ratio of the partial pressure of hydrogen to the partial pressure of carbon dioxide is beneficial for the ability of the biomass i.e. of the methanogenic microorganisms to produce methane. On the contrary, the methanogenic microorganisms require a different ratio of the partial pressure of hydrogen to the partial pressure of carbon dioxide than for methane production for cell growth, i.e. for biomass concentration increase. Thus, it was surprisingly found out by the inventor that the partial pressure ratio of hydrogen to carbon dioxide in the reaction vessel has a high impact on the performance of the cells, i.e. either mainly cell growth is promoted or mainly methane is produced.

Thus, step c) of the method of the invention preferably comprises at least one methane production phase and optionally at least one cell growth phase. Principally, the two phases are characterized by a different ratio of the partial pressure of hydrogen to the partial pressure of carbon dioxide in both phases. It is even more surprising that a ratio of the partial pressure of hydrogen to the partial pressure of carbon dioxide inside the reaction vessel which is maintained at or adjusted to 5:1 or higher (parts hydrogen : parts carbon dioxide) promotes methane production of the methanogenic microorganisms, i.e. the carbon flux from carbon dioxide is directed to methane. Preferably, the ratio of the partial pressure of hydrogen to the partial pressure of carbon dioxide in the reaction vessel in the at least one methane production phase is maintained in the range from 7:1 to 30:1 (parts hydrogen : parts carbon dioxide), more preferably, in the range from 9:1 to 25:1 (parts hydrogen : parts carbon dioxide), even more preferably, in the range from 10:1 to 18:1 (parts hydrogen : parts carbon dioxide), most preferably, the ratio of the partial pressure of hydrogen to the partial pressure of carbon dioxide inside the reaction vessel is maintained in the range from 12:1 to 17:1 or adjusted to 15±1:1 or 18±1:1 (parts hydrogen : parts carbon dioxide). "Or higher" with respect to a partial pressure ratio denotes that the ratio is increasing so that more parts hydrogen in comparison to parts carbon dioxide are present in the gas phase inside the reaction vessel, in particular more than 5 parts hydrogen are facing 1 part carbon dioxide. Examples of such "higher ratios" are also given above and elsewhere in the description.

On the contrary, a different ratio of the partial pressure of hydrogen to the partial pressure of carbon dioxide inside the reaction vessel has surprisingly been identified as promoting cell growth, i.e. biomass concentration increase, hence, wherein the carbon flux from carbon dioxide is directed to biomass production, of the methanogenic microorganisms, thus, in the at least one cell growth phase, preferably the partial pressure ratio of hydrogen to carbon dioxide is maintained at or adjusted to a ratio lower than the ratio in the at least one methane production phase, particularly preferably, the partial pressure ratio is maintained at 4:1 or lower (parts hydrogen : parts carbon dioxide) in the at least one cell growth phase, more preferably, the partial pressure ratio is maintained in the range from 0.5:1 to 4:1 or from 0.75:1 to 3.5:1 (parts hydrogen : parts carbon dioxide), even more preferably, the partial pressure ratio is maintained in the range from 1:1 to 2.5:1 (parts hydrogen : parts carbon dioxide), most preferably, the partial pressure ratio is maintained at or adjusted to a ratio of 1.5±0.5:1 or 2±1:1 (parts hydrogen : parts carbon dioxide) in the at least one cell growth phase. "Or lower" with respect to a partial pressure ratio denotes that the ratio is decreasing so that less parts hydrogen in comparison to parts carbon dioxide are present in the gas phase inside the reaction vessel, in particular less than 4 parts hydrogen are facing 1 part carbon dioxide. Examples of such "lower ratios" are given also given above and elsewhere in the description.

"Phase" or "fermentation phase" in the sense of the invention describes a situation, i.e. a condition, state or fermentation condition of the methanogenic microorganisms in the reaction vessel of the invention which is characterized by specific fermentation conditions which are applied to the methanogenic microorganism, e.g. the ratio of the partial pressures of hydrogen and carbon dioxide or the oxidation reduction potential is maintained at or in a range or adjusted to as mentioned above. Any "cell growth phase" and in particular the "at least one cell growth phase" is a phase of the invention, wherein the ratio of the partial pressure of hydrogen to the partial pressure of carbon dioxide inside the reaction vessel is maintained at or adjusted to a ratio different to the ratio in a methane production phase and thereby mainly characterized by an increase of the biomass concentration by cell division of the methanogenic microorganisms of the invention, i.e. by cell growth of the methanogenic microorganisms of the invention. Any "methane production phase" and in particular the "at least one methane production phase" is a phase of the invention, wherein the ratio of the partial pressure of hydrogen to the partial pressure of carbon dioxide inside the reaction vessel is maintained at or adjusted to 5:1 or higher (parts hydrogen : parts carbon dioxide) and thereby mainly characterized by methane production. However, during any cell growth phase, the cells may also or may not produce methane and during any methane production phase, the biomass concentration may increase, remain the same or decline, preferably, the biomass concentration increases in the methane production phase and in particular in the at least one methane production phase.

The method of the invention may further comprise an intermediate phase, wherein the methanogenic microorganisms in the reaction vessel are switched from a methane production phase, in particular from the at least one methane production phase, to a cell growth phase, in particular to the at least one cell growth phase, or from a cell growth phase, in particular from the at least one cell growth phase, to a methane production phase, in particular to the at least one methane production phase. While the methanogenic microorganisms are in an intermediate phase, they may produce methane or not or the cells may divide, grow or not.

The methanogenic microorganisms of the invention in step c) of the method are switched from one phase to another phase, i.e. from a methane production phase, in particular from the at least one methane production phase, to a cell growth phase, in particular to the at least one cell growth phase, or from a cell growth phase, in particular from the at least one cell growth phase, to a methane production phase, in particular to the at least one methane production phase, by maintaining or adjusting the ratio of the partial pressure of hydrogen to the partial pressure of carbon dioxide at or to values as disclosed above. Optionally, also the oxidation reduction potential is maintained or adjusted using a reducing agent. Any substance which reduces the oxidation reduction potential inside the reaction vessel without being harmful to the cells or having an inhibitory effect on cell growth or methane production may be used as reducing agent. Examples for such reducing agents are sodium sulphide or hydrogen sulphide. Alternatively, the oxidation reduction potential may be maintained or adjusted by the oxygen gas inflow, hydrogen inflow and/or pH shift. The oxidation reduction potential is measured in real time, for example by a redox probe from Mettler-Toledo GmbH (Greifensee, Switzerland) or any other suitable means, and therefore a specific adjustment or maintaining is possible. The reducing agent may be pulsed into the reaction vessel or fed in a continuous mode.

"Oxidation reduction potential (ORP)" or simply "reduction potential" is a measure of the tendency of a chemical species to acquire electrons and thereby be reduced. ORP is measured in volts (V), millivolts (mV), or $E_h$ (1 $E_h$ = 1 mV). Each species has its own intrinsic oxidation reduction potential; the more positive the potential, the greater the species' affinity for electrons and tendency to be reduced and *vice versa.* An aqueous solution, such as the medium of the cell culture of the invention, with a lower (more negative) oxidation reduction potential has for example a tendency to donate electrons to a newly introduced species (i.e. to be oxidized by reducing a newly introduced species). Thus, the transfer of hydrogen ions between chemical species determines the pH of an aqueous solution, the transfer of electrons between chemical species determines the oxidation reduction potential of an aqueous solution, here the oxidation reduction potential inside the reaction vessel. The oxidation reduction potential inside the reaction vessel in the at least one methane production phase may be maintained below -350 mV, preferably, in the range from -400 mV to -700 mV, more preferably, the oxidation reduction potential inside the reaction vessel is maintained in the range from -500 mV to -700 mV, most preferably, maintained at or adjusted to a value of -550±50 mV. In the at least one cell growth phase, the oxidation reduction potential inside the reaction vessel is maintained below -300 mV, preferably, in the range from -300 mV to -500 mV, even more preferably, the oxidation reduction potential inside the reaction vessel is maintained at or adjusted to a value of -350±25 mV. Thus, for promoting the switching of the methanogenic microorganisms from the at least one methane production phase to the at least one cell growth phase, the oxidation reduction potential inside the reaction vessel should optionally be maintained below -300 mV, preferably, in the range from -300 mV to -500 mV, even more preferably, the oxidation reduction potential inside the reaction vessel is maintained at or adjusted to a value of -350±25 mV. For promoting the switching of the methanogenic microorganisms from the at least one cell growth phase to the at least one methane production phase, the oxidation reduction potential inside the reaction vessel should optionally be maintained below -350 mV, preferably, in the range from -400 mV to -700 mV, more preferably, the oxidation reduction potential inside the reaction vessel is maintained in the range from -500 mV to -700 mV, most preferably, maintained at or adjusted to a value of -550±50 mV.

Surprisingly, a specific oxidation reduction potential is required to start cell growth after inoculation of a medium in the reaction vessel in step c) of the method with the methanogenic microorganisms of the invention. Here, the oxidation reduction potential should be adjusted to a value of -350±25 mV. This value may be achieved by different means, for example by adding 500 mM $Na_2SxH_2O$ to the inoculated medium. Inoculation procedures will be explained in detail below.

A methane production phase and a cell growth phase, in particular the at least one methane production phase and the at least one cell growth phase, are characterized by the different ratios of the partial pressures of hydrogen and carbon dioxide as described in detail above. The oxidation reduction potential in both phases is beneficially maintained or maintained at or adjusted to the values or in the ranges as also described in detail above; however, the ratio of the partial pressures is the main characteristic and determines the phase of the methanogenic microorganisms.

The phases of the methanogenic microorganisms, the growth phase, the intermediate phase and the methane production phase may be separated i.e. do not have to follow timely immediately after each other as the methanogenic microorganisms may be stored at lower temperatures depending on the specific cell strain used. Also, the phases do not have to occur in the same reaction vessel but the cells may be grown in one reaction vessel and be transferred to another reaction vessel for methane production. For this purpose, either two or more bioreactors may be used or the bioreactor may comprise at least two reaction vessels, at least one for cell growth and at least one for methane production. All fermentation conditions, in particular the ratio of the partial pressures of hydrogen and carbon dioxide or the oxidation reduction potential inside the reaction vessel comply according to the phase of the methanogenic microorganisms.

In a preferred set-up of step c) of the method of the invention, first, a suitable medium, e.g. standard medium, is placed in the reaction vessel and is inoculated with a suitable amount of the methanogenic microorganisms. In case the methanogenic microorganism is a methanogenic archaea, the suitable amount of cells is provided in a cell suspension which is anaerobically transferred to the reaction vessel. Anaerobic procedures and techniques can be taken from the literature, such as Sowers, Schreier *et al.*, 1995. A suitable amount of the methanogenic microorganisms of the invention is for example 0.5 to 10% of a living cell suspension of the methanogenic microorganism respective to the target volume. However, in principle a single cell is enough to inoculate the medium and thus, the amount may vary in a broad range. The skilled person knows that microorganisms and also methanogenic microorganisms grow exponentially, thus, the more cells are used for inoculation, the earlier the cells reach a certain biomass concentration. Further, inoculation procedures are known to the skilled person and may be individually selected without having a negative effect on the success of the invention (Schoenheit, Moll *et al.*, 1980).

Optionally, the at least one cell growth phase may follow. During the at least one cell growth phase, the ratio of the partial pressures of hydrogen and carbon dioxide is maintained at or adjusted to a ratio different compared to the ratio in the at least one methane production phase, preferably, the ratio of the partial pressure of hydrogen to the partial pressure of carbon dioxide in the at least one cell growth phase is maintained at or adjusted to a ratio of the partial pressure of hydrogen to the partial pressure of carbon dioxide which is lower than the ratio of the partial pressure of hydrogen to the partial pressure of carbon dioxide in the at least one methane production phase, particularly preferably, the partial pressure ratio in the at least one cell growth phase is maintained at or adjusted to 4:1 or lower (parts hydrogen : parts carbon dioxide), more preferably, the partial pressure ratio is maintained in the range from 0.5:1 to 4:1 or from 0.75:1 to 3.5:1 (parts hydrogen : parts carbon dioxide), even more preferably, in the range from 1:1 to 2.5:1 (parts hydrogen : parts carbon dioxide), most preferably, the partial pressure ratio is maintained at or adjusted to a ratio of $1.5\pm0.5$:1 or $2\pm1$:1 (parts hydrogen : parts carbon dioxide) in the at least one cell growth phase.

In general, the length of the at least one cell growth phase depends on the cell growth rate of the methanogenic microorganisms in the cell culture; however, the time period of any cell growth phase and in particular of the at least one cell growth phase may be chosen by the experimenter performing the method of the invention. In principle, the least one cell growth phase is not necessary for the success of methane production of the method of the invention as the cells may also grow and divide under the fermentation conditions of the at least one methane production phase, in particular characterized by a specific $H_2/CO_2$ partial pressure ratio. Surprisingly, it has been found out by the inventor that at least one cell growth phase is additionally beneficial for a high biomass concentration in the methane production phase which results in a higher methane yield of step c) and the overall method. This is possibly due to the surprising discovery that the partial pressure ratio which is applied in the cell growth phase promotes the carbon flux from carbon dioxide to biomass prior to enter the methane production phase, wherein the carbon flux is mainly directed from carbon dioxide to methane. Another advantage of an additional cell growth phase is that a catalytically acceptable biomass concentration of 3 g/L is reached after a shorter period of fermentation time and hence, the productivity of the overall methane production process is optimized. Hence, the method of the invention describes for the first time how - and even that it is possible at all - the carbon flux may be directed according to the needs of the experimenter and according to the procedural requirements.

Surprisingly, the inventor has further found out that it is additionally beneficial for the volumetric productivity of the overall methane production of step c) of the method of the invention to maintain the methanogenic microorganisms in the at least one cell growth phase until the cell growth of the methanogenic microorganisms in the reaction vessel stagnates (optionally, at around 3 g/L), i.e. the biomass concentration does not increase any further, or the biomass concentration reaches a value of at least 3 g biomass per litre. The biomass concentration may be determined as described elsewhere, for example gravimetrically (Schill, van Gulik *et al.*, 1996). A person skilled in the art, of course, knows other methods to determine the biomass concentration which might be used as well for this purpose.

In principle, at any time during fermentation in step c) of the method of the invention, the methanogenic microorganisms may be switched from a cell growth phase to a methane production phase or an intermediate phase or from a methane production phase to a cell growth phase or an intermediate phase by adjusting the fermentation conditions if regarded beneficial by the experimenter for example to even more increase the biomass concentration. Thus, also during stable methane production in step c) of the method of the invention, preferably in the methane production phase it may be desirable to temporarily switch the methanogenic microorganisms to cell growth phase, for example for cell growth after medium exchange or oxygen contamination. Hence, the method of the present invention may also comprise two or more methane production phases, optionally one, two or more cell growth phases and optionally one, two or more intermediate phases.

After the optional cell growth phase, the fermentation conditions of the methane production phase are applied, in particular the ratio of the partial pressures of hydrogen to carbon dioxide inside the reaction vessel is maintained at or adjusted to the ratio or in the range of the methane production phase. Thereby, the methanogenic microorganisms are switched to the methane production phase, optionally the methanogenic microorganisms pass an intermediate phase. The microorganisms suitable for the invention are maintained in this phase over a period of time which can range from hours to weeks to months and years depending on fresh medium supply, the cell species and the specific needs of the experimenter. The biomass concentration in the at least one methane production phase may increase to a value of at least 6 g biomass per litre, preferably at least 7 g biomass per litre, more preferably at least 8 g biomass per litre, even more preferably at least 9 g biomass per litre and most preferably at least 10 g biomass per litre, wherein no additional methanogenic microorganism is added after inoculation or cell retention is applied during fermentation, but the biomass concentration is reached by cell division of the originally inoculated cells which are in general 0.5 to 10% of the inoculation volume from an earlier harvest of 3 g/L stored in a pressurized bottle at +4 °C. However, a person skilled in the art knows that the number of cells which are used for inoculation has only little influence on the biomass yield that may be achieved during fermentation. It was absolutely unexpected that the experimental set-up provided by the method of the invention leads to such high biomass concentrations of the methanogenic microorganisms, in particular of the methanogenic archaea. Thus, the use of the methanogenic microorganisms of the invention inside a reaction vessel having a biomass concentration of at least 6 g biomass per litre under continuous fermentation conditions such as described herein is also provided herein.

The skilled person is well aware of the fact that in any phase of the invention, the fermentation conditions have to be adjusted to the specific needs of the employed microorganism species or cell strain; however, the fermentation conditions as described herein apply to all species and strains of the invention for cell growth and methane production.

"Biomass" or "cell culture" or "cells" in the invention equally denote to the same collective of methanogenic microorganisms of one or more species which is used in the method of the invention for converting hydrogen and carbon dioxide into methane. The methanogenic microorganisms, i.e. cells of the biomass or cell culture in the reaction vessel of the invention, principally function as a catalyst for the biological conversion of carbon dioxide and hydrogen into methane. They are placed inside the reaction vessel of the invention by inoculation of the medium inside the reaction vessel or by inoculation of the medium prior to the transfer of the medium into the reaction vessel of the invention. Thus, during fermentation the term "methanogenic microorganisms" also denotes to methanogenic microorganisms in a suspension with medium as inside a reaction vessel, the cell culture of methanogenic microorganisms typically comprises medium. The methanogenic microorganisms may be removed with or without medium from the reaction vessel either completely or partially during fermentation and in any phase for various purposes, such as exchange of cells or medium and/or for analytics, such as cell weight determination or cell vitality examination, or harvest for future inoculum. Methane is obtained from the gas leaving the liquid phase, i.e. the cell-medium suspension.

Biomass obtained from the reaction vessel may be used as source for the recovery of metabolites and as basis for new culture medium. "Healthy biomass" denotes a good vitality status of a living biomass, i.e. of the living methanogenic microorganisms. A "microorganism" is an organism that is unicellular or lives in a colony of cellular organisms. Microorganisms are very diverse; they include bacteria, fungi, archaea, and protists; microscopic plants (such as green algae); and animals such as plankton and the planarian. Some microbiologists also include viruses, but others consider these as non-living. "Methanogenic" in the sense of the invention denotes the capability of converting carbon dioxide and hydrogen into methane, i.e. being able to perform the reaction pathway of $CO_2+4H_2\rightarrow CH_4+2H_2O$ which is called methanogenesis.

A "methanogenic microorganism" is any microorganism which is capable of producing methane from other substances, i.e. capable of methanogenesis, preferably from an in-gas comprising carbon dioxide and hydrogen in various ratios, thus the methanogenic microorganisms are capable of producing methane from hydrogen and carbon dioxide. The methanogenic microorganism of the invention may be grown in a reaction vessel, which is for example comprised in a bioreactor, under controlled fermentation or cell culture conditions in a controlled environment. In principle, any such methanogenic microorganism can be used for the method of the invention. "Environment" or "controlled environment" refers to the surrounding of the methanogenic microorganisms inside the fermenter or reaction vessel comprising both, the liquid, characterized for example by the medium composition, feed and exchange rate, and the gaseous phase, characterized for example by the gas feed and gas feed composition, and the applied fermentation conditions.

The methanogenic microorganism in the cell culture of the invention is obtainable from public collections of organisms, such as the American Type Culture Collection, the Deutsche Stammsammlung für Mikroorganismen und Zellkulturen (DSMZ, Braunschweig, Germany), CBS (Utrecht, Netherlands) and the Oregon Collection of Methanogens, or they can be isolated from a variety of environmental sources. Such environmental sources include anaerobic soils and sands, bogs, swamps, marshes, estuaries, dense algal mats, both terrestrial and marine mud and sediments, deep ocean and deep well sites, sewage and organic waste sites and treatment facilities, animal intestinal tracts, volcano areas and faeces. Suitable cell cultures may be pure i.e. contain only cells of a single species, or may be mixed cultures i.e. contain cells of more than one species. Preferably, a pure cell culture of methanogenic microorganisms is used for the method of the invention.

A methanogenic microorganism by nature being capable of producing methane is especially suitable for any embodiment of the method of the invention. Preferably, the methanogenic microorganism of the invention is a methanogenic archaea, comprising all methanogenic members of the Archaeal domain, such as for example *Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arbor iphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermoautotrophicus, Methanobacterium thermoautotrophicus, Methanothermobacter thermoflexus, Methanothermobacter thermophilics, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barken, Methanosarcina mazei, Methanosarcina thermophila, Methanomicrobium mobile, Methanocaldococcus jannaschii, Methanococcus aeolicus, Methanococcus maripaludis, Methanococcus vannielii, Methanococcus voltaei, Methanothermococcus thermolithotrophicus* and *Methanopyrus kandleri*. Preferred are methanogenic archaea which belong to the species *Methanosarcinia barkeri, Methanothermobacter marburgensis,* such as for example DSM 2133 from Deutsche Stammsammlung für Mikroorganismen und Zellkulturen (DSMZ, Braunschweig, Germany), *Methanobacterium thermoautotrophicus, Methanocaldococcus jannaschii, Methanothermobacter thermoautotrophicus, Methanococcus maripaludis,* or mixtures thereof, more preferably, the methanogenic archaea and thus the preferred methanogenic microorganism is of the species *Methanothermobacter marburgensis,* most preferably, the methanogenic microorganism or methanogenic archaea is *Methanothermobacter marburgensis* strain DSM 2133. "Archaea" are single-celled microorganisms. Archaea have no cell nucleus or any other membrane-bound organelles within their cells. In the past they were viewed as an unusual group of bacteria and named archaeabacteria, however, to date archaea are considered as having an independent evolutionary history since they show many differences in their biochemistry in comparison to other forms of life. Thus, they are now classified as a separate domain (Archaeal domain) in the three-domain system. In this system the phylogenetically distinct branches of evolutionary descent are the archaea, bacteria and eukarya.

In principle, the methanogenic microorganism in the invention, i.e. the biomass, is used in cell culture, i.e. the methanogenic microorganism is used as suspension with medium. The medium of the invention comprises at least a source of nitrogen, assimilable salts, a buffering agent and trace elements. Sulphur is provided to the cells by the reducing agent or may be provided extra, thus, the reducing agent and the sulphur-providing substance may or may not be the same. Sulphur may be provided to the cells by the provision of biogas. The medium is generally designed to keep the cells vital, healthy and productive without being expensive and thus beneficial for the entire process economy of the method of the invention. Prior to inoculation of the medium with the methanogenic microorganism of the invention, the medium should be degassed for being anaerobic as is required for optimal methane production by the methanogenic microorganism which is used in the particular experiment, this applies in particular if the methanogenic microorganism is a methanogenic archaea. The skilled person knows techniques to prepare an oxygen free medium, for example by flushing the medium with a gas mixture of 80% hydrogen and 20% carbon dioxide (v/v = volume per volume) or with nitrogen for 5 min per litre medium. Standard medium compositions may be taken from the literature and be adapted (Schoenheit, Moll *et al.*, 1980; Schill, van Gulik *et al.*, 1996; Liu, Schill *et al.*, 1999). An example for a standard medium has the following composition ($L^{-1}$): 2,1 g $NH_4Cl$; 6,8 g $KH_2PO_4$; 3,4 g $Na_2CO_3$; 0,09 g Titriplex I; 0,04 g $MgCl_2$x $6H_2O$; 0,01 g $FeCl_2$x$4H_2O$; 0,2 mg $CoCl_2$x$6H_2O$; 1,2 mg $NiCl_2$x$6H_2O$; 0,2 mg $NaMoO_4$x$2H_2O$; the pH will be discussed below and can be adjusted by titrating 1 M $(NH_4)_2CO_3$, NaOH or $NH_4OH$. Said exemplary standard medium may be used for any embodiment of the method of the invention.

Independent from the phase of the methanogenic microorganisms in the cell culture, the medium or single components of the medium are refreshed in a constant or stepwise mode during fermentation under continuous conditions. The feed rate or infeed rate of medium or medium components is generally adjusted between 0.001 $h^{-1}$ and 0.1 $h^{-1}$. The out-flow rate corresponds to the in-flow rate plus water which is produced by the methanogenic microorganisms during methanogenesis. Said water can be re-used for various purposes, such as medium preparation or else.

The skilled person is aware that the medium has to be adjusted to the specific needs of the microorganism species, i.e. cell strain. In general, the fermentation conditions, i.e. medium composition, and other parameters, such as $H_2/CO_2$ partial pressure ratio, pH, temperature, stirring speed, pressure, oxidation reduction potential or medium or medium component (i.e. consumables) feed rate, i.e. fresh medium supply rate, have to be adjusted according to the specific needs of the microorganism strain selected and the procedural requirements depending for example on the phase of the methanogenic microorganisms in the reaction vessel. Herein, information is provided which particular fermentation condition is applicable to all methanogenic microorganisms, such as the standard medium, stirring speed, feed rate, oxidation reduction potential, pressure, or the partial pressure ratios of hydrogen and carbon dioxide, and which should be adapted to the specific needs of a cell species, such as the temperature and pH value.

"Consumable" comprises all substances, components and/or materials in any form, i.e. solid, gaseous or liquid, which might be required by the methanogenic microorganisms in the reaction vessel of the invention, in any phase of the fermentation, e.g. in the methane production phase, the intermediate phase, or in the cell growth phase. Also included are substances which are fed into the reactor to maintain a certain fermentation condition, such as a certain medium composition, a specific oxidation reduction potential or partial pressure ratio. The supply of consumables can be adjusted by the experimenter throughout the method of the invention and in any phase of the methanogenic microorganism if deemed appropriate by the experimenter independent from the phase of the methanogenic microorganisms. "Procedural requirements" comprise all situations which may arise during the method of the invention in any phase of the methanogenic microorganism, such as the requirement to switch to another phase or in response to oxygen contamination, feeding real gas, or else. The method of the invention is designed for the conversion of hydrogen and carbon dioxide into methane by methanogenic microorganisms in a reaction vessel; however, it might be necessary for a high volumetric productivity to switch from the methane production phase to an intermediate or cell growth phase, wherein excess liquid, parts of the gas phase or the biomass are partially or completely exchanged. Such phases may be used for example to increase the cell density of the cell culture inside the reactor or to introduce fresh medium or medium components. At no time, the overall feasibility of the invention is impeded.

"Off-gas", "exhaust gas", "outgas" or "output gas" denote the gaseous outcome of the reaction vessel of the invention and thus of the method which is typically a gas mixture leaving the reaction vessel. The qualitative and quantitative content of the off-gas depends on various factors, such as the phase of the methanogenic microorganisms in the reaction vessel, the total gas feed rate, and the composition of the in-gas. The off-gas may comprise water vapour, the gases comprised in the in-gas, such as for example hydrogen, carbon dioxide or hydrogen sulphide, and gases which are produced by the methanogenic microorganisms such as methane. In all phases of the method of the invention, the off-gas mixture may further contain contaminants which are in the in-gas or originate from the cell culture, for example oxygen, compounds from biogas generation, and others. During methane production phase, the off-gas mixture mainly comprises methane, preferably the methane content is at least 40%, 45%, 50%, or 60%, more preferably the methane content is at least 70%, more preferably 80%, 84%, 86%, or 88%, even more preferably the methane content in the off-gas is at least 90%, most preferably the methane content is at least 92%, 94% or 96% wherein the higher the content the more preferred. During cell growth phase, the methane content in the off-gas is typically between 50% and 80%. The methane from the off-gas may be separated by standard means, e.g. by membranes such as obtainable from Du Pont (Wilmington, DE, USA) or Gore (Newark, DE, USA). It may further be used in end-user applications as energy carrier for energy applications to produce heat and/or electric energy or else or as raw material in various chemical and biological conversions.

"In-gas" denotes any gas or gas mixture which is fed into the reaction vessel and thus provided to the methanogenic microorganism. The in-gas or in-gas feed comprises the gases which are required for the production of methane, i.e. hydrogen and carbon dioxide, and other gases which may be required for other purposes such as for example to adjust the oxidation reduction potential in the reaction vessel by addition of hydrogen sulphide or are required for other biological processes of the microorganisms or which are introduced as contaminants. This is especially the case if real gases are used as gas source for the method of the invention. "Feed" means the introduction or transfer of a gas, liquid, suspension or any other substance into the reaction vessel or into the bioreactor of the invention. "In-gas feed" denotes the feed or flow or introduction of the in-gas, such as hydrogen or carbon dioxide, into the reaction vessel of the invention. "Hydrogen in-gas feed" denotes the hydrogen comprising gas or in-gas which is introduced into the reaction vessel or bioreactor and "carbon dioxide in-gas feed" denotes the carbon dioxide comprising gas or in-gas which is introduced into the reaction vessel or bioreactor.

"Real gas" denotes that the gas is not absolutely pure, i.e. is gas mixture. In case of hydrogen and carbon dioxide, "real gas" denotes that beside carbon dioxide or hydrogen also other gases are comprised which are denoted as contaminants. On the contrary, an "ideal gas" is absolutely pure according to general industrial standards. A typical example for a real gas is "biogas" or also the off-gas of the method of the invention. "Biogas" typically refers to a gas produced by the biological breakdown of organic matter, e.g. biomass, in the absence of oxygen, such as for example biomass fermentation. Biogas originates from biogenic material and is a type of biofuel like bioethanol. Biogas is produced by anaerobic digestion or fermentation of biodegradable materials, i.e. of biomass, such as manure, sewage, municipal waste, green waste, plant material and energy crops. Thus, "biomass fermentation" denotes the anaerobic digestion or fermentation of biodegradable materials, i.e. of biomass. This type of biogas comprises primarily methane and carbon dioxide and is preferred as carbon dioxide real gas source of the invention. Other gas species generated by use of biomass is wood gas, which is created by gasification of wood or other biomass. This type of gas consists mainly of nitrogen, hydrogen, and carbon monoxide, with trace amounts of methane.

In general, an "industrial process" is any process which involves chemical or mechanical steps to aid in the manufacture of an item or items, usually carried out on a larger scale by man and not by nature. For the present invention, relevant industrial processes produce carbon dioxide and/or hydrogen or other gases and substances which might be required for the method of the invention either as main product or as side product. If said substances are a side product, they are sometimes referred to as waste products (e.g. "industrial waste gas"). Industrial processes which produce or release hydrogen or carbon dioxide are also denoted as "industrial sources" for both gases. Depending on the purity of the released gas, the gas is referred to as being an "ideal gas" having a very high or absolute purity/quality or being a "real gas" having a lower or not absolute purity, meaning that one or more other gases may be present in various amounts, however, the gas species not being harmful to the cells of the invention or being present in the real gas in an amount not being harmful to the cells.

The carbon dioxide which is used for the method of the invention and is converted into methane by the methanogenic microorganism of the invention may be pure or of high quality/purity, i.e. "ideal gas", or "real gas", characterized in that it comprises beside carbon dioxide also other gases which are denoted as contaminants. In general, the carbon dioxide may be of any source. These carbon dioxide sources include but are not limited to ideal gas sources delivering ideal gas or real gas sources delivering real gas. Ideal gases may be obtained by various purchasers which are known to the skilled person (e.g. Air Liquide, Paris, France). Sources of real gas are natural sources, such as the atmosphere, fixed carbon dioxide in living or dead biomass or industrial processes, also denoted as "industrial sources", comprising the combustion or oxygen combustion or oxygen enriched combustion of carbonaceous material, such as biomass, waste, biofuel, fossil fuels, or else, the burning of vegetable matter, biogas, bioethanol, biomass fermentation or anaerobic digestion to produce liquid fuels and coal, biomass gasification processes, general gasification, any other carbon dioxide releasing process, carbon dioxide contained in the off-gas of the method of the invention or combinations thereof. Preferably, carbon dioxide real gas is used for the method of the invention, i.e. carbon dioxide from a real gas source, more preferably the carbon dioxide real gas is from an industrial process, such as industrial waste gas, biogas or biomass fermentation, off-gas of the method of the invention itself, from other methanogenesis performed by methanogenic archaea, oxygen combustion or oxygen enriched combustion or mixtures thereof. In particular, using the off-gas of the method of the invention itself and using carbon dioxide which was produced by oxygen enriched combustion using the oxygen produced by the electrolysis of water in step b) of the method of the invention improves educt utilization, economy and cost-effectiveness of the overall method. Real gas of carbon dioxide or hydrogen is often cheaper and also makes a contribution to environmental protection due to optimal raw material usage. And carbon dioxide, which would otherwise be released to the atmosphere as waste gases and potentially would contribute to global warming, is recycled and used as new energy source raw material. "Combustion", e.g. "oxygen enriched combustion" or "oxygen combustion", is a process wherein carbonaceous material, e.g. petroleum, coal, living and/or dead biomass reacts with an oxidizing element, such as oxygen, into carbon dioxide or also carbon monoxide, by thermal conversion. The oxygen which is required for combustion may be derived from the electrolysis of water (see below). Devices for combustion, oxygen enriched combustion or oxygen combustion may be obtained from Mitsubishi Heavy Industries, Tokyo, Japan. Biomass for combustion and/or gasification may also be the methanogenic microorganisms of the invention which are dried as it might be advisable to completely empty the bioreactor, remove the methanogenic microorganisms from the reaction vessel and start methanogenesis with a completely new batch of cells.

Carbon dioxide of any source and also mixtures thereof may be purified from contaminants prior to its feed into the reaction vessel of the bioreactor of the invention by various methods which are known to the person skilled in the art, such as using a carbon dioxide scrubber, contacting the gas with an absorbing medium which selectively absorbs carbon dioxide, such as amine, monoethanolamine solutions or quicklime absorption, or which selectively absorbs gases other than carbon dioxide from the real gas, activated charcoal or activated coal, by the regenerative carbon dioxide removal system, polymer membrane gas separators, molecular sieves, others and combinations thereof. All aforementioned methods are also useful for recovery, purification, enriching, storing, recycling and/or further processing of carbon dioxide which is contained in the off-gas, and thus was not converted into methane by the methanogenic microorganisms, of the method of the invention. After carbon dioxide is separated from other components of the off-gas it may be fed back by tubes or other devices and means which are known to the skilled person into the reaction vessel comprised in an in-gas and is thus recycled. In this way, the use of carbon dioxide is optimized. In general, carbon dioxide of any natural, industrial, technical or artificial process, which releases carbon dioxide, may be used as well as carbon dioxide from gasification. "Gasification" is a process which converts carbonaceous materials, e.g. petroleum, coal, living and/or dead biomass, biofuel or else into carbon dioxide, hydrogen and carbon monoxide, by thermal conversion and a controlled amount of oxygen and/or steam into a resulting gas mixture called synthesis gas or syngas. In equilibrium, the concentrations of carbon monoxide, water vapour, carbon dioxide and hydrogen balance. In principle, a limited amount of oxygen or air is introduced into the reactor (= device) to allow some of the organic material to be "burned" to produce carbon monoxide and energy, which drives a second reaction that converts further organic material to hydrogen and additional carbon dioxide. Further reactions occur when the formed carbon monoxide and residual water from the organic material react to form methane and excess carbon dioxide. Catalysts can be used to improve the reaction rates. The oxygen which is required for gasification may be derived from the electrolysis of water (see below). Preferably, the carbon dioxide comprising real gas is purified according to any of the techniques mentioned above or known in the art, to increase the carbon dioxide content to around 80%. Gasification reactors or devices can be obtained from Condens Heat Recovery Oy (Hämeenlinna, Finland), Babcock & Wilcox Vølund A/S (Esbjerg, Denmark) or Siemens AG (Munich, Germany).

Carbon dioxide, i.e. carbon dioxide gas, of any source, e.g. ideal or real, may be used during cell growth, methane production and intermediate phase of the invention. In general, carbon dioxide of different ideal and real sources may be mixed in any ratio and the mixture may be used in any phase of the invention. It is also appropriate to mix the gas sources or change the gas sources during fermentation or during performing the method. It is an advantage of the invention that the carbon dioxide and hydrogen content in the gas sources may vary greatly without affecting the feasibility of the invention. This is due to the novel and inventive approach of the invention to determine the partial pressures of carbon dioxide and hydrogen in real time during fermentation and adjust or maintain the pressure ratio inside the reaction vessel to or at a specific value or range the pressure ratio inside the reaction vessel to a specific value in a range according to the desired carbon flux, i.e. towards cell growth in the cell growth phase or towards methane production in the methane production phase. It is not even necessary to determine the exact composition of the real gas or determine the exact hydrogen content in the real gas which is another very important advantage of the method of the invention over the methods known in the art.

The hydrogen, i.e. hydrogen gas, which is used for the method of the invention and may be converted into methane by the methanogenic microorganisms of the invention may be pure or of high quality/purity, i.e. "ideal gas", or "real gas" characterized in that it comprises beside hydrogen also other gases which are denoted as contaminants. In general, the hydrogen may be of any source. Ideal hydrogen gases may be obtained by various purchasers which are known to the skilled person (e.g. Air Liquide, Paris, France). Hydrogen sources include but are not limited to ideal gas sources delivering ideal gas, or real gas sources delivering real gas, such as natural sources, industrial processes (industrial "waste gas") comprising the electrolysis of water or brine, propane dehydrogenation, hydrogen from oil refining, such as from cracking process, biomass fermentation, be made via methane steam reforming or hydrogen may be off-gas released from the method or reaction vessel of the invention or from other methanogenesis performed by methanogenic archaea. Hydrogen is recovered from the off-gas of the method or reaction vessel of the invention using a separation device such as a membrane which allows the comparatively small hydrogen molecules to pass through the pores of the membrane and allows the comparatively large molecules of the other gas components to be rejected by the membrane. Such separation devices and membranes may be obtained for example from Du Pont (Wilmington, DE, USA) or Gore (Newark, DE, USA). The hydrogen recovered from the off-gas is then fed back into the reaction vessel via tubes or means which are known to the skilled person to be used as hydrogen source for the conversion into methane by the methanogenic microorganisms of the invention. The use of hydrogen of the off-gas of the invention improves educt utilization and process economy of the overall method. In general, hydrogen of different sources may be mixed in any ratio and the mixture may be used in any phase of the invention. As it also applies for carbon dioxide, it is also appropriate to mix the hydrogen gas sources or change the hydrogen gas sources during fermentation or during performing the method as also the partial pressures of hydrogen is determined in real time during the method and a determined ratio of the partial pressure of hydrogen to the partial pressure of carbon dioxide is adjusted or maintained in real time inside the reaction vessel. Thus, the hydrogen content in the gas sources may vary greatly without affecting the feasibility of the invention, preferably, the hydrogen comprising real gas comprises at least 80% of hydrogen, more preferably at least 85%, even more preferably at least 90%, most preferably at least 95%, wherein it might be necessary to purify the hydrogen comprising real gas prior to use in the method of the invention according to any of the techniques mentioned above or known in the art. Also for hydrogen real gas, it is not necessary to determine the exact composition of the real gas or determine the exact hydrogen content in the real gas which is also a very important advantage of the method of the invention over the methods known in the art. "Electrolysis" in the sense of the invention means a method or process which uses an electric current to induce an otherwise non-spontaneous chemical reaction. In the process, an electric current passes through a substance, thereby causing a chemical change of said substance, usually the gaining or losing of electrons. Electrolysis requires an electrolytic cell, such as an electrolyser, e.g. Hofmann voltameter, consisting of separated positive and negative electrodes (anode and cathode, respectively) immersed in an electrolyte solution containing ions or in a molten ionic compound. Reduction occurs at the cathode, where electrons are added that combine with positively charged cations in the solution. Oxidation occurs at the anode, where negatively charged anions give up electrons. Standard or high-pressure electrolyser can be obtained from various manufacturers such as from Hydrogen Technologies (Notodden/Porsgrunn, Norway), Proton Energy Systems (Wallingford, CT, USA), Heliocentris Energy Solutions AG (Berlin, Germany), Claind (Lenno, Italy), Hydrogenics GmbH (Gladbeck, Germany), Sylatech Analysentechnik GmbH (Walzbachtal, Germany), h-tec Wasserstoff-Energie-Systeme GmbH (Luebeck, Germany), ze-botec GmbH (Konstanz, Germany), $H_2$ Logic (Herning, Denmark), QuinTech (Goeppingen, Germany), and electrolysis may be performed according to the manufacturer's instructions. In the electrolysis of water, water is used as the substance through which the electronic current passes. The electronic current leads to the decomposition of the water into oxygen ($O_2$) and hydrogen ($H_2$). The overall reaction equation is: $2\ H_2O(\text{liquid}) \rightarrow 2\ H_2(\text{gaseous}) + O_2(\text{gaseous})$. Hydrogen can also be produced by the electrolysis of brine (i.e. a water sodium chloride mixture). This electrolysis is commonly used for the production of chlorine, wherein hydrogen is produced as a side product. Herein, the passed through current leads to the oxidation of chloride ions to chlorine. The overall reaction equation is: $2\ NaCl + 2\ H_2O \rightarrow Cl_2 + H_2 + 2\ NaOH$. The water and/or brine for the electrolysis may be obtained by any source, such as tap water, from rivers, lakes, sea water, rain or waste water from industrial processes (here the explanations of industrial waste gas apply accordingly). In case that the water and/or brine does not have the necessary purity for the electrolysis, the water and/or brine may be purified by distillation, filtration and/or centrifugation and other means which are well known to a person skilled in the art and can for example be taken from "Water Treatment, Principles and Design", 2. edition, 2005, John Wiley & Sons.

The energy for any industrial process mentioned herein which provides hydrogen, oxygen, carbon dioxide or any other precursor for the method of the invention, in particular for the electrolysis of water or brine to provide hydrogen and/or oxygen which may then be used for gasification or combustion processes to provide carbon dioxide, may principally be provided by any energy source, such as renewable or non-renewable energy sources, such as electricity from combustion of fossil fuels and related energy carriers or nuclear energy. Preferably, the energy for the industrial processes is from a renewable energy source, in particular the energy for the hydrogen-producing electrolysis, for gasification or combustion is from a renewable energy source. "Renewable energy" is energy which comes from natural resources or sources and is renewable, i.e. naturally replenished. Renewably energy comprises wind power, solar power, geothermal power, water/hydro power, wave power, tidal power, biofuels, biomass and combinations thereof. More preferably, the energy source is selected from the group consisting of solar, wind, water and geothermal power. A "device for generating electric energy from a renewable and/or non-renewable energy source" are the reactors or devices, i.e. respective power plants, which convert the energy of the energy source, such as thermal, kinetic, chemical and/or mechanical energy into electric energy. Said power plants may be large-scale power plants or household-scale devices or else.

Solar powered electrical generation relies on concentrated solar heat (e.g. heat engines) and solar power (e.g. photovoltaics in solar panels). A device for generating electric energy from solar power may for example be selected from photovoltaics, concentrated solar power system or else and may be obtained for example from Bosch Solar Energy AG (Erfurt, Germany), sonnen_systeme Projektgesellschaft mbH (Alheim-Heinebach; Germany), First Solar (Tempe, USA), Suntech (Wuxi, China), Sharp (Tokyo, Japan), Q-Cells (Bitterfeld-Wolfen, Germany) and Stirling Energy Systems, Inc. (Scottsdale, AZ, USA). Solar hot water system solar heat engines may be used for heating the bioreactor of the system.

Biomass is also regarded as renewable energy source and is biological material from living or recently living organisms, such as wood, waste, (hydrogen) gas, and alcohol fuels. Living or recently living organisms comprise plants and trees, such as miscanthus, switchgrass, hemp, corn, poplar, willow, sorghum, sugarcane, eucalyptus or palms, but also forest residues, e.g. dead trees, branches and tree stumps, yard clippings, wood chips, garbage, plant or animal matter used for production of fibres or chemicals, and biodegradable waste that can be burnt as fuel. Excluded are such organic materials as fossil fuels which have been transformed by geological processes into substances such as coal or petroleum. Biofuels, e.g. bioethanol, biobutanol (biogasoline), biogas, syngas, bioethers, biodiesel or solid biofuels as wood pellets, cube or pucks, are a wide range of fuels which are in some way derived from biomass. The energy for the production of biomass or biofuel is thus derived from the sun and converted by photosynthesis into the biological material. Devices for generating electric energy from biomass or biofuel comprise biomass power plants, and can be obtained for example from SBBiogas GmbH (Marktbreit, Germany), Evonik New Energies GmbH (Saarbruecken, Germany) or Envi Con & Plant Engineering GmbH (Nuremberg, Germany).

A device for generating electric energy from wind power may for example be selected from wind turbine, wind mill, wind pump or drainage. Suppliers of said devices are for example for small applications: Superwind (Bruehl, Germany), Braun Windturbinen (Nauroth, Germany), Urban Green Energy (New York, USA) and Helix Wind (Poway, USA). Exemplary suppliers of wind turbines for larger applications are Vestas (Randers, Denmark), GE Wind Energy (Fairfield, USA), Sinovel (Beijing, China), Enercon (Aurich, Germany), Goldwind (Urumqui, China), Gamesa (Zamudio, Spain) and Siemens Wind Power (Brande, Denmark).

Geothermal electricity is electricity generated from geothermal energy. A device for generating electric energy from geothermal power may for example be selected from dry steam power plant, flash steam power plant, hybrid plant and binary cycle power plant and may be obtained for example from Ansaldo Energia (Genoa, Italy) or ECONAR (Maple Grove, MN, USA).

In general, the production of hydro electricity uses the gravitational force of falling or flowing water. Conventional hydroelectric power stations use the potential energy of dammed water for driving a water turbine and generator. The energy amount obtained in this way depends on the water volume and the difference in height between the source and the water's outflow. Pumped-storage hydroelectric power stations produce electric energy to supply high peak demands by moving water between reservoirs of different levels. Here, water is released back into the lower reservoir through a turbine which is connected to a generator or preferably may be connected to the bioreactor or device for producing hydrogen and/or oxygen by the electrolysis of water and/or brine. Also, run-of-the-river hydroelectric stations, small, micro or pico hydro stations are available. A device for generating electric energy from water/hydro power may for example be obtained from AC-Tec G.m.b.H (Kaltern, Italy) or EnBW Energie Baden-Wüfttemberg AG (Karlsruhe, Germany).

Wave power denotes the energy of ocean surface waves. A device for generating electric energy from wave power may for example be selected from point absorber or buoy, surfacing following or attenuator oriented parallel to the direction of wave propagation, terminator, submerged pressure differential, oriented perpendicular to the direction of wave propagation, oscillating water column, or an overtopping facility. The devices comprise a hydraulic ram, elastomeric hose pump, pump-to-shore, hydroelectric turbine, air turbine or a linear electrical generator, optionally also a parabolic for increasing the wave energy at the point of capture. Such devices may be located close to shoreline, near shore or offshore. Suppliers are for example Ocean Navitas Ltd. (Marton, UK), AWS Ocean Energy Ltd. (Inverness, UK), BioPower Systems (Mascot, Australia), Columbia Power Technologies (Corvallis, OR, USA), Ocean Power Technologies (Pennington, NJ, USA) or Pelamis Wave Power (Edinburgh, UK).

A device for generating electric energy from tidal power which uses the daily rise and fall of water due to tides may for example be selected from tidal power stations, undershot waterwheels, dynamic tidal power plants, tidal barrage, tidal lagoons and turbine technology, such as axial turbines or crossflow turbines. Suppliers are for example BioPower Systems (Mascot, Australia), Neptune Renewable Energy Ltd. (North Ferriby, UK) or Clean Current Power Systems Incorporated (Vancouver, Canada).

Carbon dioxide and hydrogen are both required for methane production. Both gases are generally fed into the reaction vessel of the invention according to the desired ratio of their partial pressures. They may be pure or contaminated with other gases tolerated by the system, i.e. not irreversibly harmful to the methanogenic microorganisms. Inside the reaction vessel, the gases are generally dissolved in the medium of the invention which comprises the methanogenic microorganisms and are taken up by the microorganisms of the invention and converted into methane, subsequently. The gases may be fed separately into the reaction vessel or be mixed outside the reaction vessel in a ratio which results in a certain partial pressure ratio and then fed in a mixture. The mix-ratio may be adjusted in real time according to the determined partial pressure ratio of hydrogen and carbon dioxide inside the reaction vessel as described above. The gas feed rates of each gas may vary between 0.05 vvm (vvm = gas volume per liquid volume and minute) and 5 vvm, preferably the gas feed rate is between 0.1 vvm and 3 vvm, even more preferably between 0.2 vvm and 2 vvm, most preferably the gas feed rate is between 0.4 vvm and 1 vvm. The liquid volume is determined gravimetrically, by differential pressure or radar or any other method known to the skilled person.

Typical "contaminants" of the gases which are fed into the reaction vessel of the invention are process dependent and may be oxygen, chlorine, carbon monoxide, nitrogen and others. In response to oxygen contamination of the reaction vessel, the oxidation reduction potential has to be stabilized using sulphide or else, adjusting the hydrogen in-gas feed and the pH value, wherein the concrete action may be chosen by the person skilled in the art.

A "fermentation condition" or "phase condition" is a parameter of the method of the condition which may be adjusted by the skilled person who performs the method of the invention. In general, "fermentation conditions" in the sense of the invention are all standard fermentation conditions which are known to a person skilled in the art of microorganism fermentation (Bailey, Ollis (1986) Biochemical Engineering Fundamentals, McGraw-Hill Science, New York, USA). Examples of fermentation conditions are the partial pressures of hydrogen and carbon dioxide and thus the ratio of the partial pressures of hydrogen and carbon dioxide, the oxidation reduction potential, the pH value, biomass concentration, temperature, pressure, stirring speed, medium composition, gas flow rates, medium exchange rates and others. The skilled person knows that in a reaction vessel containing a suspension of living organisms such as the methanogenic microorganisms of the invention the fermentation conditions, which are required for optimal cell performance regarding cell growth or methane production, may fluctuate and vary and will have to be adjusted accordingly by the experimenter. Since all relevant fermentation conditions may be measured or determined either in real time or by simple means, the skilled person knows how to the fermentation conditions can be adjusted to successfully perform the method of the invention.

The cell growth phase and the methane production phase are characterized by a different ratio of the partial pressure of hydrogen to the partial pressure of carbon dioxide inside the reaction vessel. Optionally, also the oxidation reduction potential is different in both phases, values are given above. The $H_2/CO_2$ partial pressure ratios and the optional different oxidation reduction potential of both phases apply to all methanogenic microorganisms of the invention, preferably to the methanogenic archaea, described herein. The optimal fermentation temperature and pH depend on the cell species and microorganism strain used and information thereon will be given in detail below. All other fermentation conditions apply to both phases and to all species of methanogenic microorganisms, i.e. have no significant effect on cell growth or methane production of the methanogenic microorganisms.

The $H_2/CO_2$ partial pressure ratio may be adjusted or maintained according to the real time measurement of the reaction vessel. It is possible to feed both gases separately in the amount which is suitable to adjust or maintain a specific ratio inside the reaction vessel or to mix both gases prior to the feed into the reaction vessel in a ratio which results in the desired ratio of the partial pressures of carbon dioxide and hydrogen inside the reaction vessel. The ratio of the partial pressures of hydrogen to the partial pressure of carbon dioxide may be determined in real time as described above.

"Tolerated" means that something which is not directly having a beneficial and/or positive effect on the process of electrolysis, generating electric energy or the methanogenic microorganism, the reaction vessel, the experimental set-up of the method of the invention as a whole, the composition of the in-gas or off-gas and/or the methane production rate of the invention but still does not lead to methane production inefficiency or overall process inefficiency of the method of the invention or prevents the success of the method of the invention or the use of the methanogenic microorganism.

The temperature inside the reaction vessel depends on the methanogenic microorganism which is used for the method of the invention. In general, for all methanogenic archaea the temperature should be in the range from 40 °C to 100 °C. Preferably, for mesophilic microorganisms, such as *Methanosarcinia barkeri* or *Methanococcus maripaludis,* the temperature of the biomass in the reaction vessel is in the range of about 30 °C to about 40 °C, more preferably in the range of about 35 °C to about 37 °C. Preferably, for thermophiles such as *Methanothermobacter marburgensis, Methanobacterium thermoautotrophicus* or *Methanothermobacter thermoautotrophicus* the temperature inside the bioreactor was about 60 °C to 67 °C, preferably about 65±1 °C, or about 85 °C to 90 °C for organisms such as *Methanocaldococcus jannaschii.*

The stirring speed is adjusted to a value which is optimal for a high gas liquid mass transfer in the volume of the respective reaction vessel, in a typical fermentation the stirring speed is at least 20 rpm or 50 rpm, preferably, at least 500 rpm, more preferably at least 1000 rpm, even more preferably at least 1500 rpm, most preferred, the stirring speed is at least 1500 rpm or the maximum which can be applied by the stirrer used in the reaction vessel of the bioreactor. Thus, the stirring speed in particular of large-volume reaction vessels, such as 15,000 L, may be significantly less than 1500 rpm, for example in the range between 20 rpm to 100 rpm, e.g. 65 rpm.

The pH of the cell culture inside the reaction vessel should be broadly in the neutral range. Preferably, for all methanogenic microorganisms of the invention the pH value of the invention is in the range of 5.0 to 8.0, more preferably, the pH value is in the range of 5.5 to 7.8, even more preferably, the pH value is in the range of 6.4 to 7.6, in particular preferably, the pH value is in the range of 6.7 to 7.2, most preferably, the pH value is about 6.9±0.5.

In general, methanogenesis is an anaerobic process and inhibited by oxygen. However, certain amounts of oxygen may be tolerated by the system. If the oxygen content is too high, for example a common oxygen scrubber may be used to remove oxygen from the in-gas. Experiments, for example in WO 2008/094282, have shown that even 100% oxygen feed for more than 10 hours only stopped methane production for a that time and that the system soon recovered and started to again produce methane. In no way, the system will be permanently damaged. Thus, the system is very robust to oxygen contamination and oxygen is tolerated to a certain extend. It is, however, preferred to have a very low oxygen contamination inside reaction vessel, preferably below 2%.

Inside the reaction vessel, the pressure may be adjusted to a value in the range from 1 bar to 500 bar absolute, preferably the pressure may be between 1 bar and 300 bar absolute, even more preferably, the pressure may be between 1 bar and 200 bar, most preferably the pressure may be between 1 bar and 100 bar absolute.

During the whole fermentation procedure, i.e. during any phase of the method, it is appropriate to adapt the fermentation conditions, such as the stirring speed, the temperature, the supply rate of any liquid, gaseous or solid compound, the pressure and/or pH value in order to change the fermentation conditions inside the bioreactor comprising the reaction vessel for various purposes including but not limited to the increase of methane production and/or of cell growth. Classically, two fermentation modes are applicable in the methods described herein (in the literature also denoted as phases although herein not to be mixed up with the "phases of the invention" characterized by a specific $H_2/CO_2$ partial pressure ratio): the batch mode (= batch cultivation mode or batch fermentation mode) and the continuous mode (=continuous cultivation mode or continuous fermentation mode). In the batch mode, typically no mass (solid, liquid, gaseous) transfer into the reaction vessel or into the bioreactor is allowed. Thus, during batch mode the system comprising the bioreactor and the methanogenic cells in the reaction vessel is closed. The only exception is that hydrogen and carbon dioxide may still be fed as in-gases into the reaction vessel and be provided to the methanogenic microorganisms inside the reaction vessel or not. If this is the case, the fermentation mode may also be regarded as semi-batch mode. On the contrary, the continuous cultivation mode is characterized by a continuous supply with fresh medium or medium components, gases or other substances which are continuously required by the cells in the reaction vessel, such as hydrogen, carbon dioxide or medium. In general, the reaction volume, i.e. the culture volume, inside the reaction vessel is kept constant, so that also gaseous and/or liquid substances have to be removed from the reaction vessel depending on the total mass in-flow. Thus, during the continuous mode, the system comprising the bioreactor and the methanogenic cells in the reaction vessel can be regarded as being open and components are exchanged.

Hence, these two fermentation modes describe a setting of the bioreactor comprised in the system. Batch mode conditions are very often applied during the initial phase when cells are dividing and biomass is increasing exponentially, whereas continuous cultivation conditions are often applied when cell growth stagnates or is constant. In this mode, the cells are usually kept over a longer period of time for producing the desired product, here methane.

During a methane production phase or a cell growth phase of step c) of the method of the invention characterized by the partial pressure ratios of hydrogen and carbon dioxide as described above, the fermentation mode may be batch mode or continuous mode. If the cells are only kept in the methane production phase according to the partial pressure ratio of hydrogen and carbon dioxide, batch and continuous conditions may be applied as regarded beneficial by the experimenter, however, the inventor found out that best results may be achieved when the mode is switched from batch to continuous when cell growth stagnates in the methane production phase. If at least one cell growth phase and at least one methane production phase are applied, it was found out that it is especially beneficial if the batch mode conditions are applied during cell growth phase and the continuous cultivation mode is applied during methane production phase. The switch between both phases and mode is then performed as described above when cell growth stagnates or reaches at least a concentration of 3 g biomass per litre.

The bioreactor (= fermenter or reactor) comprises all components and devices which are necessary to perform step c) of the method of the invention, i.e. to produce methane of hydrogen and carbon dioxide. For this purpose, the bioreactor comprises at least a reaction vessel suitable for fermenting, growing and culturing the methanogenic microorganisms of the invention, devices for the supply of liquid, solid and gaseous substances and for analytics. "Suitable for fermenting, growing and culturing the methanogenic microorganisms" in the sense of the invention denotes that the reaction vessel allows to maintain a surrounding or condition which promotes cell growth, fermentation and culturing of the cells. This comprises, for example, maintaining the culture at a pre-determined temperature, stirring the cells and providing substances which are essential for the metabolism of the cells, such as gases, medium or medium components. In principle, the reaction vessel may be any container wherein methanogenic microorganism may be cultured, grown and fermented. In general, the reaction vessel is made of glass or steel depending on the pressure which is applied to the culture of methanogenic microorganisms. The reaction vessel may be connected to all necessary devices and measuring probes for adjusting the fermentation conditions of the invention to grow the methanogenic microorganisms up to a desired biomass concentration and/or to switch the phase of the methanogenic microorganisms or to keep the cells in one of the phases over a period of time which may be selected from the experimenter. The period of time may be understood as minutes or hours or days or even longer. The method of the invention also allows keeping the methanogenic microorganisms in a non-methane-producing state without further cell growth.

The bioreactor suitable for the invention may be derived from a standard bioreactor suitable for culturing, fermenting, growing and/or processing of a biomass comprising the methanogenic microorganisms of the invention. Suppliers of standard bioreactors are, for example, Applikon Biotechnology B.V. (Schiedam, The Netherlands), Infors (Bottmingen, Switzerland), Bioengineering (Wald, Switzerland) and Sartorius Stedim Biotech GmbH (Göttingen, Germany).

The bioreactor, i.e. reactor comprises at least one vessel or container suitable for culturing, fermenting and/or processing of the methanogenic microorganisms, i.e. the reaction vessel of the invention. The bioreactor may also comprise two or more reaction vessels, one or more for cell growth and also one or more for methane production. Even different ratios of the partial pressure of hydrogen to carbon dioxide may be applied in the reaction vessels which are used for the same phase of the invention. These reaction vessels may be linked, for example via tubes, pipes or else, to transport the cell culture comprising the suspension of methanogenic microorganisms from one reaction vessel to the other or back if needed. Said one or more reaction vessels may also not be directly linked to each other, in this case, the methanogenic microorganism may be transferred from one reaction vessel to the other via another container. The bioreactor of the invention may be equipped with various means and devices for adjusting and monitoring the fermentation conditions in the bioreactor and in the reaction vessel or in the reaction vessels. With reference to the bioreactor of the invention, "suitable for the method" denotes that the bioreactor comprises the components and devices which are necessary to culture the microorganisms of the invention in a way that they are able to grow and produce methane and what else is necessary to perform the method of the invention.

In particular, the bioreactor is suitable for the method of the invention and comprises:

a) at least one reaction vessel for fermenting, growing and/or culturing methanogenic microorganisms,
b) at least one device for introducing and removing gaseous substances into the at least one reaction vessel,
c) at least one device for introducing or removing a liquid substance or a cell suspension into or from the bioreactor or reaction vessel,
d) devices for measuring and/or adjusting at least the oxidation reduction potential, temperature, pressure, head pressure, off-gas concentration and content, the partial pressure ratio of hydrogen and carbon dioxide and/or pH value,
e) at least one device for removing off-gas from the at least one reaction vessel,
f) at least one stirring device inside the at least one reaction vessel, and/or
g) at least one device for gas liquid mass transfer, such as an agitator.

Optionally, the bioreactor comprises a device for a continuous feed of medium and/or reducing agent and/or sulphur source.

Optionally, the bioreactor comprises at least one device for the gas feed of carbon dioxide and a separate device for the gas feed of hydrogen. In one exemplary embodiment, an ideal stirred tank bioreactor is used for the method of the invention. This bioreactor comprises a reaction vessel with head plate and multiple inlet and outlet ports. The probes for temperature, pH and oxidation reduction potential measurement are fitted to the ports. The bioreactor is further equipped with an agitator with impeller blades (Rushton) on at least three levels. Medium and liquid reducing agent is fed via a pump, controlled gravimetrically or with a flow meter or is needed to control the oxidation reduction potential. Acid or base is added by titration to control pH as desired. "Gas liquid mass transfer" denotes the transfer of mass in general, i.e. molecules and atoms, between a liquid and a gaseous phase, wherein phase is to be understood in its thermodynamic meaning and not to be mixed up with a fermentation "phase of the invention". Of particular importance for the method of the invention is the transfer of hydrogen and carbon dioxide from the gaseous phase to the liquid phase comprising the biomass suspension and the methanogenic microorganism inside the reaction vessel, and the transfer of methane from the liquid phase to the gaseous phase inside the reaction vessel to leave the reaction vessel. The gas liquid mass transfer indirectly influences the mass transfer from the liquid to the solid phase, such as the biomass.

The bioreactor and also the reaction vessel comprised in the bioreactor may be connected to various sources of hydrogen and carbon dioxide, nitrogen, real gases and others through the at least one device for introducing of gaseous substances and further to automated systems for feeding liquid, gaseous and solid substances and materials into the bioreactor via peristaltic pumps and others. Also devices and means may be attached to the bioreactor which measure the composition of the in-gas and which further improve the quality of the in-gas, in case of a real gas application, or the off-gas. The bioreactor also comprises a device for removing and measuring the off-gas from the reaction vessel. The off-gas composition, in particular the amounts of hydrogen, carbon dioxide and methane, may be measured for example by an individually applied gas analyser system, such as supplied by BlueSens gas sensor GmbH (Herten, Germany). The off-gas produced by the method of the invention may optionally be further processed by standard means, such that hydrogen and carbon dioxide, which were not converted into methane, are separated from methane and or from each other. Hydrogen and carbon dioxide which are comprised in the off-gas are preferably recycled in that they are fed back into the reaction vessel of the invention. Hydrogen may be separated from the off-gas of the invention using a separation device such as a membrane which allows the small hydrogen molecules to pass through the pores of the membrane and does not allow the passage of larger molecules or by classical condensation using a cold finger or else. Aforementioned methods are of course also applicable to increase the hydrogen content in the used hydrogen educt gas or real gas. Carbon dioxide may be separated from the off-gas of the invention for example with an absorbing medium which selectively absorbs carbon dioxide or which selectively absorbs gases other than carbon dioxide from the off-gas or by any method described above. Both hydrogen and carbon dioxide which have been recovered from the off-gas may be used as educts in the method of the invention. After or before or while hydrogen and carbon dioxide are removed from the off-gas also other gases such as water vapour and other gases may be removed to increase the percentage of methane in the off-gas for methane or natural gas based applications. The higher the percentage of methane, and thus the purity, in the off-gas, the more preferred. Most preferred is a percentage of methane in the off-gas which closely resembles natural gas, i.e. is between 40% and 90%, preferably between 60% and at least 90%.

The bioreactor may further comprise devices which allow the sterile and anaerobic entry and exit of substances required for the method of the invention. "Substances required for the method of the invention" are for example the biomass comprising the methanogenic microorganisms, the medium or medium components, water, gases, such as hydrogen, carbon dioxide, methane, hydrogen sulphide, nitrogen or mixtures thereof, and compositions which might contain a mixture of all these substances.

The bioreactor suitable for the invention is further equipped with discharge lines, pumps and compressors. The bioreactor at least comprises all devices which are necessary to feed the gases which are required for the method of the invention, i.e. at least hydrogen and carbon dioxide, liquid substances, such as the cell culture suspension and fresh medium or medium components, devices for removing the off-gas of the invention, excess liquid and gaseous parts from inside the bioreactor, and all other components which are necessary to control the fermentation parameters of the invention.

In a preferred embodiment, the bioreactor is suitable for culturing, fermenting and/or processing of a biomass comprising microorganisms under high pressure, e.g. a pressure higher than the normal atmospheric pressure. For this purpose, the bioreactor comprises a reaction vessel or housing made of a material which is suitable for high pressure and fulfils the requirements of occupational safety and follows work safety rules. The material of the housing may be selected from the group comprising steel, such as stainless steel according to European standard EN 10088, for example 1.43XX, 1.44XX, 1.45XX, or else.

A reaction vessel of the bioreactor suitable for the invention for culturing methanogenic microorganisms may have different volumes, wherein larger volumes are preferred for the industrial production of methane. The industrial production of methane also comprises the methane production in decentralized household applications, wherein the volume of the reaction vessel comprised in the bioreactor may be in the smaller and middle range, and largely depends on the particular methane amount which is needed and the available space. In any embodiment of the invention, the volume of the reaction vessel is at least 1 L, preferably at least 5 L, 10 L, 25 L, more preferably at least 50 L, 100 L, 500 L, 1000 L, even more preferably the volume of the reaction vessel is at least 5000 L, 10,000 L, 20,000 L, 50,000 L, most preferably the volume of the reaction vessel is at least 100,000 L. In another preferred embodiment, more than one reaction vessel is comprised in the bioreactor of the invention or more than one bioreactor comprising one or more reaction vessels are combined, for example attached serially. In a serial arrangement, the volume of the reaction vessel is typically smaller, i.e. in the range of 0.5 L to 20 L, preferably in the range of 1 L to 15 L, more preferably in the range of 1 L to 10 L and most preferably in the range of 1 L to 5 L, however, if regarded beneficial, the volume of the reaction vessel may also be larger, as indicated above, for example at least 1 L, preferably at least 5 L, 10 L, 25 L, more preferably at least 50 L, 100 L, 500 L, 1000 L, even more preferably the volume of the reaction vessel is at least 5000 L, 10,000 L, 20,000 L, 50,000 L, or the volume of the reaction vessel is at least 100,000 L. Such a serial arrangement allows using the hydrogen, methane and carbon dioxide comprising off-gas as in-gas for the next following reaction vessel and bioreactor. Optionally, the methane is removed from the off-gas before it is re-used as in-gas. Thereby, the process economy is improved and methanogenesis educts, i.e. hydrogen and carbon dioxide, which have not been converted into methane in the preceding reaction vessel, may again be used. Of course, in each reaction vessel, the fermentation conditions and in particular the $H_2/CO_2$ partial pressure ratio and thus the phase of the methanogenic microorganisms may be applied individually. It is also possible to use different methanogenic species and mixtures thereof in each of the reaction vessels.

In a preferred embodiment of step c) of the method of the invention, the bioreactor is designed in a way that the carbon dioxide and the hydrogen released from the reaction vessel in the off-gas of the invention is re-used in the method of the invention. Thus, the hydrogen and carbon dioxide of the off-gas of the invention are recycled.

**BRIEF DESCRIPTION OF THE DRAWINGS**

Figure 1:  General set-up of the system. The system comprises at least one device for generating electric energy from a renewable and/or non-renewable energy source (1), at least one device for producing hydrogen and/or oxygen by the electrolysis of water and/or brine (2), preferably an electrolyser, and at least one bioreactor (3) comprising a reaction vessel suitable for growing, fermenting and/or culturing methanogenic microorganisms for converting hydrogen and carbon dioxide into methane. Optionally, the renewable energy source comprises solar energy, wind power, wave power, tidal power, water/hydro power, geothermal energy, biomass and biofuel combustion. Especially for initiating the system, it might also be necessary to introduce energy from outside, i.e. from a generator or from the common public power supply system. Arrows indicate the transfer of one or more compounds (e.g. electric energy, liquid, gaseous and/or solid compounds) between the devices/bioreactor(s) or else of the system. The electric energy from a renewable and/or non-renewable energy source may be transferred via general power supply lines or cables or else to the at least one device for producing hydrogen and/or oxygen by the electrolysis of water and/or brine (2), preferably to an electrolyser, and/or to the at least one bioreactor (3) and/or optionally be at least partially stored in an electric energy storage device (4), such as a battery or accumulator or else. In general and applicable to all embodiments of the invention, the electric energy storage device (4) may be any common device suitable for storing electric energy, such as a common battery or common accumulator to be used according to the manufacturer's instructions. From the electric energy storage device (4) the electric energy may be transferred via general power supply lines or cables or else to the at least one device for producing hydrogen and/or oxygen by the electrolysis of water and/or brine (2), preferably to an electrolyser, and/or to the at least one bioreactor (3). Water and/or brine are provided via general transportation or tubes, pipes or else but also via water tanks transported by transportation systems such as trains, cars, trucks or else. Water in general, and in particular water from rain or water which was purified in order to be suitable for the use in electrolysis may be stored in water tanks, cisterns, artificial lakes, reservoirs or else. The hydrogen obtained by electrolysis is transferred to the bioreactor (3), where it is converted together with carbon dioxide into methane by methanogenic microorganisms. Hydrogen and carbon dioxide may be transferred to the bioreactor via standard gas transport means, such as tubes, pipes or else. Both may also be delivered, for example, in gas cylinders or bottles which allow for storage of carbon dioxide and/or hydrogen before introduction into the bioreactor. Gas cylinders, storage cylinders, storage vessels and/or bottles not only allow storage of carbon dioxide or of hydrogen after electrolysis in case of excess production or else prior to introduction into the bioreactor, but also allow individual transportation of carbon dioxide and/or hydrogen to bioreactors which are - for example - not connected to any gas transport means, such as tubes, pipes or else or storage or transportation of the off-gas or methane prior to end-user applications. The off-gas is removed from the bioreactor via the standard means of the

**EP 2 675 904 B2**

bioreactor, such as tubes, pipes or else and may be transported directly to end-user applications via tubes, pipes or else or in gas cylinders, storage cylinders, storage vessels and/or bottles or be further processed as described elsewhere in the description. These standard means are familiar to a person skilled in biotechnology and can also be taken from the bioreactor manufacturer's instructions. The off-gas comprises methane, water vapour, hydrogen and carbon dioxide but also other gases which are present inside the reaction vessel as described below. Preferably, said bioreactor further comprises at least one device for measuring the head pressure and the off-gas concentration and at least one device for adjusting or maintaining the partial pressure ratio of hydrogen to carbon dioxide inside the reaction vessel. Please note that the size or thickness of any symbol, arrow, font or else is not intended to express yields, importance or conversion rates.

Figure 2: Exemplary set-up of the system which illustrates examples for the further use of the off-gas of the invention which is released from the bioreactor/reaction vessel. By standard gas transportation means as described herein (tubes, pipes, gas cylinders/bottles or else) the off-gas in transferred to one or more devices for recovery, purification, enriching, storing, recycling and/or further processing of off-gas (6). These devices (6) may be combined or used individually in any embodiment of the system of the invention described herein and comprise for example devices for increasing the content of methane in the off-gas by removing other substances contained in the off-gas, such as for example hydrogen or carbon dioxide, and/or remove methane from the off-gas. Thereby, methane is purified and also enriched for various end-user applications (7), wherein methane is used as energy carrier for thermal, mechanical, electric energy or else and/or wherein methane is converted into diverse base chemicals, such as methanol, olefin or gasoline. Exemplary devices for all purposes are described in detail above and in the description. Devices for storing off-gas, methane, carbon dioxide or hydrogen may be gas tanks, cylinders, bottles or any other suitable vessel or reservoir. The off-gas, hydrogen and/or carbon dioxide may be fed back into one or more bioreactors (3). In general, methane optionally burns with oxygen, such as comprised in air, ideal or real gas or produced by electrolysis of water, to carbon dioxide, which may be re-used for methane production in the bioreactor by the methanogenic microorganisms, and water, which may be re-used for electrolysis for hydrogen and oxygen production. Optionally, also a device for oxygen enriched combustion or gasification may be present. For all other components or devices of the exemplary embodiment of the system of the invention depicted herein, for example (1), (2), (3) or (5) please refer to the explanation of Figures 1, 3 or 4. Of course, also here optionally an electric energy storage device (4), such as a battery or accumulator or else, may be present. Further, all components or intermediates may be stored in gas tanks, cylinders, bottles or any other suitable vessel or reservoir until further use or for transportation reasons. Please note that the size or thickness of any symbol, arrow, font or else is not intended to express yields, importance or conversion rates. Of course, this exemplary embodiment may comprise the features depicted in Figure 1 or 3 or any other embodiment described herein and in the description. All components shown in the Figure may be individually selected by the person skilled in the art and combined or left away.

Figure 3: Exemplary set-up of the system comprising at least one device for generating electric energy from a renewable energy source (1), at least one device for producing hydrogen and/or oxygen by the electrolysis of water and/or brine (2), preferably an electrolyser, and at least one bioreactor (3) comprising a reaction vessel suitable for growing, fermenting and/or culturing methanogenic microorganisms for converting hydrogen and carbon dioxide into methane. Optionally, the renewable energy source comprises solar energy, wind power, wave power, tidal power, water/hydro power, geothermal energy, biomass and biofuel combustion. Especially for initiating the system, it might also be necessary to introduce energy from outside, i.e. from a generator or from the common public power supply system. Arrows indicate the transfer of one or more compounds (e.g. electric energy, liquid, gaseous and/or solid compounds) between the devices or bioreactor(s) or else of the system. The electric energy from the renewable energy source, may be transferred via general power supply lines or cables or else to the at least one device for producing hydrogen and/or oxygen by the electrolysis of water and/or brine (2), preferably to an electrolyser, and/or to the at least one bioreactor (3) and/or optionally be at least partially stored in an electric energy storage device (4), such as a battery or accumulator or else. In general and applicable to all embodiments of the invention, the electric energy storage device (4), such as a battery or accumulator or else may be any common device suitable for storing electric energy, such as a common battery or common accumulator to be used according to the manufacturer's instructions. From the electric energy storage device (4) the electric energy may be transferred via general power supply lines or cables or else to the at least one device for producing hydrogen and/or oxygen by the electrolysis of water and/or brine (2), preferably to an electrolyser, and/or to the at least one bioreactor (3). Water and/or brine are provided via general transportation or tubes, pipes or else but also via water tanks transported by transportation systems such as trains, cars, trucks or else. Water in general, and in particular water from rain or water which was purified in order to be suitable for the use

24

in electrolysis may be stored in water tanks, cisterns, artificial lakes, reservoirs or else. The hydrogen obtained by electrolysis is transferred to the bioreactor (3), where it is converted together with carbon dioxide into methane by methanogenic microorganisms. Hydrogen and carbon dioxide may be transferred to the bioreactor via standard gas transport means, such as tubes, pipes or else. Both may also be delivered, for example, in gas cylinders or bottles which allow for storage of carbon dioxide and/or hydrogen before introduction into the bioreactor. Gas cylinders, storage cylinders, storage vessels and/or bottles not only allow storage of carbon dioxide or of hydrogen after electrolysis in case of excess production or else prior to introduction into the bioreactor, but also allow individual transportation of carbon dioxide and/or hydrogen to bioreactors which are - for example - not connected to any gas transport means, such as tubes, pipes or else or storage or transportation of the off-gas or methane prior to end-user applications. The off-gas is removed from the bioreactor via the standard means of the bioreactor, such as tubes, pipes or else and may be transported directly to end-user applications via tubes, pipes or else or in gas cylinders, storage cylinders, storage vessels and/or bottles or be further processed as described elsewhere in the description. These standard means are familiar to a person skilled in biotechnology and can also be taken from the bioreactor manufacturer's instructions. The off-gas comprises methane, water vapour, hydrogen and carbon dioxide but also other gases which are present inside the reaction vessel as described elsewhere in the description. Preferably, said bioreactor further comprises at least one device for measuring the head pressure and the off-gas concentration and at least one device for adjusting or maintaining the partial pressure ratio of hydrogen to carbon dioxide inside the reaction vessel. Please note that the size or thickness of any symbol, arrow, font or else is not intended to express yields, importance or conversion rates.

Figure 4: Exemplary set-up of the system further comprising at least one device for oxygen enriched combustion or gasification (5) for producing carbon dioxide. Preferably, the device for oxygen enriched combustion (5) or gasification (5) uses the oxygen produced by the electrolysis of water in device (2) for producing carbon dioxide, said carbon dioxide being transferred to the bioreactor (3), thereby being transferred to the methanogenic microorganisms inside the reaction vessel comprised in the bioreactor. Optionally, additional carbon dioxide from any other carbon dioxide source mentioned herein may be introduced into the bioreactor (3). For combustion or oxygen enriched combustion or gasification, any fuel and/or carbonaceous material, e.g. petroleum, wood, coal, living and/or dead biomass, is required and may be delivered via standard transportation as known to a person skilled in the art. For all other components of the exemplary embodiment of the system of the invention depicted herein please refer to the explanation of Figures 1 to 3. Of course, also here optionally an electric energy storage device (4), such as a battery or accumulator or else, may be present. Further, all components or intermediates may be stored in gas tanks, cylinders, bottles or any other suitable vessel or reservoir until further use or for transportation reasons. Please note that the size or thickness of any symbol, arrow, font or else is not intended to express yields, importance or conversion rates.

Figure 5: Volumetric productivity as a function of the partial pressure ratio of hydrogen to carbon dioxide in batch cultivations of *M. marburgensis*.

Figure 6: Biomass yield per mol carbon dioxide as a function of the partial pressure ratio of hydrogen to carbon dioxide in batch cultivations of *M. marburgensis*.

Figure 7: Volumetric productivity as a function of time and switch of a *M. marburgensis* culture from cell growth phase to methane production phase.

Figure 8: Biomass yield per mol carbon dioxide as a function of time and switch of a *M. marburgensis* culture from cell growth phase to methane production phase.

Figure 9: Volumetric productivity as a function of time during an equilibrium state of *M. marburgensis* in methane production phase and in continuous mode.

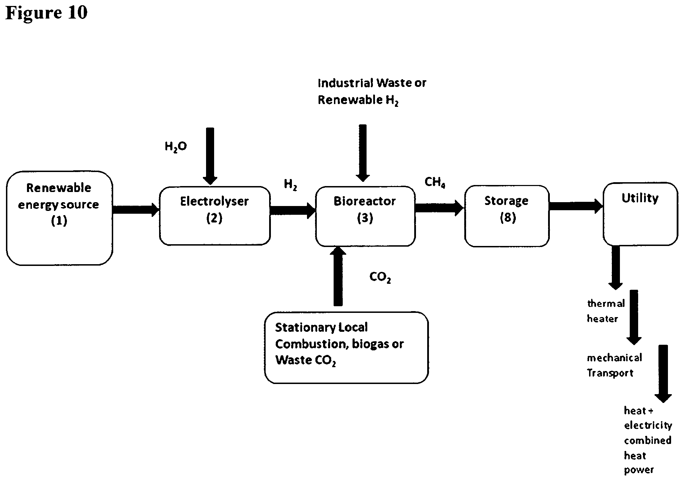

Figure 10: Exemplary set-up of the system which illustrates examples for the further use of the off-gas of the invention which is released from the bioreactor/reaction vessel. The system comprises at least one device for generating electric energy from a renewable energy source (1), at least one device for producing hydrogen and/or oxygen by the electrolysis of water and/or brine (electrolyser, 2), and at least one bioreactor (3) comprising a reaction vessel suitable for growing, fermenting and/or culturing methanogenic microorganisms for converting hydrogen and carbon dioxide into methane. Optionally, the renewable energy source comprises solar energy, wind power, wave power, tidal power, water/hydro power, geothermal energy, biomass and biofuel combustion. In this particular embodiment, industrial waste or $H_2$ from a renewable source and stationary local combustion, biogas or waste $CO_2$ are used as $H_2$ and $CO_2$ for running the methane production in the bioreactor (3). Optionally, a gas storage device (8) is used between the bioreactor (3) and the end user application (utility). Examples of end-user applications may be thermal heater, mechanical transport and/or heat + electricity combined heat power. Other aspects and features are as described in Figures 1 to 4, adapted if necessary.

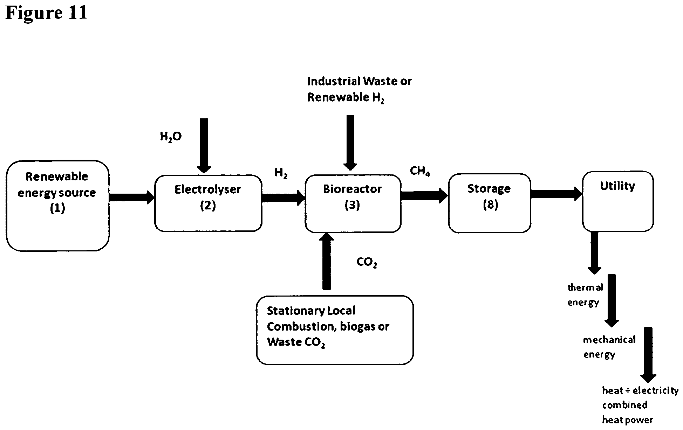

Figure 11: Exemplary set-up of the system which illustrates examples for the further use of the off-gas of the invention which is released from the bioreactor/reaction vessel. The system comprises at least one device for generating electric energy from a renewable energy source (1), at least one device for producing hydrogen and/or oxygen by the electrolysis of water and/or brine (electrolyser, 2), and at least one bioreactor (3) comprising a reaction vessel suitable for growing, fermenting and/or culturing methanogenic microorganisms for converting hydrogen and carbon dioxide into methane. Optionally, the renewable energy source comprises solar energy, wind power, wave power, tidal power, water/hydro power, geothermal energy, biomass and biofuel combustion. In this particular embodiment, industrial waste or $H_2$ from a renewable source and stationary local combustion, biogas or waste $CO_2$ are used as $H_2$ and $CO_2$ for running the methane production in the bioreactor (3). Optionally, a gas storage device (8) is used between the bioreactor (3) and the final utility such as thermal energy, mechanical energy and/or heat + electricity combined heat power. Other aspects and features are as described in Figures 1 to 4, adapted if necessary.

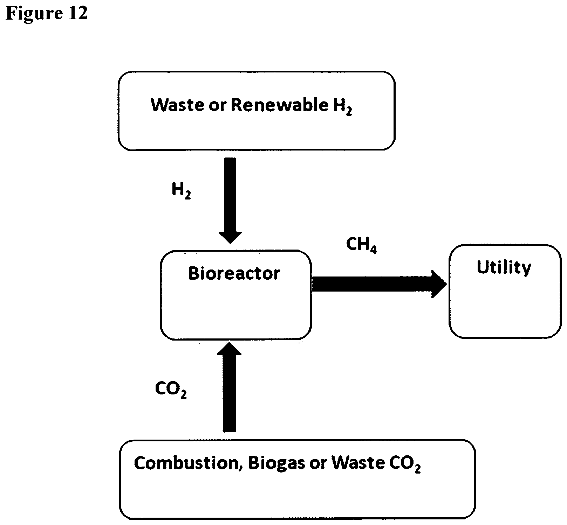

Figure 12: Bioreactor with supplied $H_2$ and $CO_2$ is shown and connection to utility devices.

## EXAMPLES

### 1. Strain and Preculture

*Methanothermobacter marburgensis* strain DSM 2133 was obtained from the Deutsche Stammsammlung für Mikroorganismen und Zellkulturen (DSMZ, Braunschweig, Germany). *M. marburgensis* was cultivated as described below and stored as a living culture at 4 °C with 0.5 bar overpressure of 80%/20% (v/v) $H_2/CO_2$ (Air Liquide, Paris, France) in pressure resistant bottles (Schott AG, Mainz, Germany) sealed with natural gum rubber stopper (Staint-Gobain, Charny, France) for maintenance of anaerobic conditions. Gas phase was replaced weekly by evacuating the gas phase from the bottle headspace and replacing with 0.5 bar of 80%/20% (v/v) $H_2/CO_2$. Strain maintenance was carried out in an anaerobic chamber flushed with formation gas (95% $N_2$, 5% $H_2$ v/v).

### 2. Cultivation Medium

The standard medium for cultivation comprised the following components ($L^{-1}$): 2.1 g $NH_4Cl$, 6.8 g $KH_2PO_4$, 3.4 g $Na_2CO_3$, 0.09 g Titriplex I, 0.04 g $MgCl_2x6H_2O$, 0.01 g $FeCl_2x4H_2O$, 0.2 mg $CoCl_2x6H_2O$, 1.2 mg $NiCl_2x6H_2O$ and 0.2 mg $NaMoO_4x2H_2O$. Prior to the transfer of the cell medium with or without cells to the reaction vessel comprised in the bioreactor, the complete bioreactor system with all devices and connected tubings etc. was made anaerobic by flushing with 80%/20% (v/v) $H_2/CO_2$ for five minutes.

### 3. Standard Fermentation without cell growth phase in 1 L scale

Cultivation of *M. marburgensis* was performed in standard medium in a 1 L Applikon bioreactor (Applikon Biotechnology B.V., Schiedam, The Netherlands) at 65°C. For inoculation, a 50 mL suspension of a living culture of any strain *M. marburgensis* was anaerobically transferred per litre of medium into the bioreactor using anaerobic procedures as described elsewhere (Sowers, Schreier *et al.*, 1995). Anaerobic 500 mM $Na_2Sx9H_2O$ was used as sulphur source and was either pulsed into the bioreactor by syringe or fed continuously via peristaltic pump at a flow rate of 1.0 mL/h to 3 mL/h in the bioreactor.

The pH value was measured via pH probe (Mettler-Toledo GmbH, Greifensee, Switzerland) and kept constant at 6.8 using 1 M $(NH_4)_2CO_3$ as base in order to compensate for acidification of the medium during growth of *M. marburgensis*. Oxidation reduction potential (ORP) was measured via redox probe (Mettler-Toledo GmbH, Greifensee, Switzerland) and kept below -350 mV. The individual gas flow of $H_2$ and $CO_2$ (Air Liquide, Paris, France) was controlled via mass flow controllers (Brooks Instrument, Matfield, USA). The total gassing rate ($H_2$ and $CO_2$) was controlled by mass flow controllers. Off-gas was detected via individually serially applied gas analyzer systems (BlueSens gas sensor GmbH, Herten, Germany) for $H_2$, $CO_2$ and $CH_4$, respectively. Biomass was determined as dry weight using standard drying procedures and gravimetric determination of preweighted glass tubes. Optical density was measured using a spectrophotometer (Hitachi U-1100, Japan) at an absorption of 578 nm. For maintainance of anaerobic conditions of the medium and of the $Na_2Sx9H_2O$ solution, a nitrogen blanket of approx. 0.1 bar was applied to each storage vessel.

The in-flow rates of the gases were regulated so that the ratio of the partial pressures of hydrogen to carbon dioxide is maintained at 5:1 or higher. For this purpose, the off-gas concentration of both gases is measured in real time and the partial pressure of each gas is determined in relation to the total pressure inside the reaction vessel, i.e. in relation to the head pressure.

For continuous culture mode of *M. marburgensis,* the medium feed supply was controlled gravimetrically via PID controller to maintain a constant feed rate.

The volume inside the reaction vessel was held constant via a harvest pump connected to an immersion pipe at a constant reactor volume of 1.0 L. The concentration of methane in the off-gas was between 60% and 90%.

Example 3 was also performed as described above but with the process variations as shown in following Table 2:

Table 2: Process variations. [1]Anaerobic 500 mM $Na_2Sx9H_2O$, feed-rate [mL/h];

| No. | In-flow rates [vvm][1] | Partial pressure ($H_2/CO_2$) | Oxidation reduction potential (mV) | $Na_2S*9H_2O$ feed-rate (mL/h)[1] | Dissolution rate [vvh][2] | Volumetric productivity [mmol/L*h][3] |
|---|---|---|---|---|---|---|
| 1 | 0,41 | 5,31 | -475 | 0,600 | 0,101 | 165 |
| 2 | 0,23 | 19,74 | -485 | 0,433 | 0,080 | 104 |
| 3 | 0,41 | 5,25 | -478 | 0,600 | 0,102 | 165 |
| 4 | 0,38 | 5,44 | -477 | 0,600 | 0,094 | 165 |
| 5 | 0,38 | 5,61 | -476 | 0,567 | 0,094 | 164 |
| 6 | 0,36 | 5,97 | -477 | 0,533 | 0,089 | 159 |
| 7 | 0,30 | 5,78 | -446 | 0,167 | 0,105 | 127 |
| 8 | 0,31 | 6,43 | -452 | 3,733 | 0,108 | 129 |
| 9 | 0,32 | 5,19 | -446 | 1,867 | 0,074 | 131 |
| 10 | 0,32 | 6,50 | -453 | 0,100 | 0,031 | 128 |
| 11 | 0,31 | 9,56 | -460 | 1,200 | 0,056 | 116 |
| 12 | 0,31 | 9,21 | -459 | 1,667 | 0,058 | 116 |
| 13 | 0,31 | 10,74 | -491 | 4,267 | 0,061 | 118 |
| 14 | 0,31 | 9,30 | -495 | 4,333 | 0,062 | 114 |
| 15 | 0,31 | 7,51 | -480 | 3,800 | 0,058 | 113 |
| 16 | 0,31 | 7,61 | -482 | 2,867 | 0,060 | 113 |
| 17 | 0,26 | 9,04 | -414 | 1,000 | 0,112 | 106 |
| 18 | 0,26 | 9,04 | -415 | 0,933 | 0,111 | 106 |
| 19 | 0,26 | 9,07 | -416 | 0,933 | 0,112 | 106 |
| 20 | 0,31 | 9,42 | -430 | 1,233 | 0,107 | 109 |
| 21 | 0,31 | 8,48 | -433 | 1,367 | 0,111 | 109 |
| 22 | 0,31 | 5,41 | -422 | 1,100 | 0,203 | 90 |
| 23 | 0,42 | 21,09 | -501 | 0,567 | 0,091 | 154 |

[2]Dissolution rate which denotes the exchanged volume per total volume per day [vvd]; [3]Volumetric productivity [mmol/L/h].

The concentration of methane in the off-gas was between 40% and 90%.

### 4. Standard Fermentation without cell growth phase in 10 L scale

Cultivation of *M. marburgensis* was performed in standard medium in a 10 L bioreactor type Biostat® Cplus (Sartorius Stedim Biotech GmbH, Göttingen, Germany) at 65°C. For inoculation, a 50 mL suspension of a living culture of *M. marburgensis* was anaerobically transferred per litre of medium into the bioreactor using anaerobic procedures as described elsewhere (Sowers, Schreier *et al.*, 1995). Anaerobic 500 mM $Na_2Sx9H_2O$ was used as sulphur source and was fed continuously via peristaltic pump at a flow rate of 7.0 mL/h to 14.0 mL/h in the bioreactor.

The pH value was measured via pH probe (Mettler-Toledo GmbH, Greifensee, Switzerland) and kept constant at 6.8 using 1 M $(NH_4)_2CO_3$ as base in order to compensate for acidification of the medium during growth of *M. marbur-*

*gensis.* Oxidation reduction potential (ORP) was measured via redox probe (Mettler-Toledo GmbH, Greifensee, Switzerland) and kept below -350 mV. The individual gas flow of $H_2$ and $CO_2$ (Air Liquide, Paris, France) was controlled via mass flow controllers (Brooks Instrument, Matfield, USA). The total gassing rate ($H_2$ and $CO_2$) was controlled by mass flow controllers. Off-gas was detected via individual serially applied gas analyzer systems (BlueSens gas sensor GmbH, Herten, Germany) for $H_2$, $CO_2$ and $CH_4$, respectively. Biomass was determined as dry weight using standard drying procedures and gravimetric determination of preweighted glass tubes. Optical density was measured using a spectrophotometer (Hitachi U-1100, Japan) at an absorption of 578 nm. For maintainance of anaerobic conditions of the medium and of the $Na_2Sx9H_2O$ solution, a nitrogen blanket of approx. 0.1 bar was applied to each storage vessel.

The inflow rates of the gases were regulated so that the ratio of the partial pressures of hydrogen to carbon dioxide is maintained at 5:1 or higher. For this purpose, the off-gas concentration of both gases is measured in real time and the partial pressure of each gas is determined in relation to the total pressure inside the reaction vessel, i.e. in relation to the head pressure.

For continuous culture mode of *M. marburgensis,* the medium feed supply was controlled gravimetrically via PID controller to maintain a constant feed rate.

The volume inside the reaction vessel was held constant via a harvest pump connected to an immersion pipe. Therefore, the reactor weight was kept constant over a feedback regulation on the harvest pump. The concentration of methane in the off-gas was between 60% and 90%.

## 5. Standard Fermentation with cell growth phase in 1 L scale

Cultivation of *M. marburgensis* was performed in standard medium in a 1 L Applikon bioreactor (Applikon Biotechnology B.V., Schiedam, The Netherlands) at 65°C. For inoculation, a 50 mL suspension of a living culture of any strain, for example of *M. marburgensis,* was anaerobically transferred per litre of medium into the bioreactor using anaerobic procedures as described elsewhere (Sowers, Schreier *et al.*, 1995). Anaerobic 500 mM $Na_2Sx9H_2O$ was used as sulphur source and was either pulsed into the bioreactor by syringe or fed continuously via peristaltic pump at a flow rate of 1.0 mL/h to 3 mL/h in the bioreactor.

The pH value was measured via pH probe (Mettler-Toledo GmbH, Greifensee, Switzerland) and kept constant at 6.8using 1 M $(NH_4)_2CO_3$ as base in order to compensate for acidification of the medium during growth of *M. marburgensis.* Oxidation reduction potential (ORP) was measured via redox probe (Mettler-Toledo GmbH, Greifensee, Switzerland). The individual gas flow of $H_2$ and $CO_2$ (Air Liquide, Paris, France) was controlled via mass flow controllers (Brooks Instrument, Matfield, USA). The total gassing rate ($H_2$ and $CO_2$) was controlled by mass flow controllers. The in-flow rates of the gases were regulated depending on the determined partial pressure ratios, Initially, during the cell growth phase, the partial pressure ratio of hydrogen to carbon dioxide was set to be maintained in the range from 0.5:1 to 4:1 (parts hydrogen : parts carbon dioxide). Within this range from 0.5:1 to 4:1, the partial pressure ratio may fluctuate. In the cell growth phase, the fermentation, and therefore also the settings of the bioreactor, was started in batch cultivation mode with an ORP value below -300 mV.

In the methane production phase, the partial pressure ratio of hydrogen to carbon dioxide was set to be maintained at 5:1 or higher. For the methane production phase, the continuous culture mode was used for long term stable operation with an ORP value below -350 mV. For a continuous culture of *M. marburgensis,* the medium feed supply was controlled gravimetrically via PID controller to maintain a constant feed rate.

The volume inside the reaction vessel was held constant via a harvest pump connected to an immersion pipe at a constant cell culture volume of 1.0 L.

During the whole fermentation procedure, off-gas was detected via individually serially applied gas analyzer systems (BlueSens gas sensor GmbH, Herten, Germany) for $H_2$, $CO_2$ and $CH_4$, respectively. Biomass was determined as dry weight using standard drying procedures and gravimetric determination of preweighted glass tubes. Optical density was measured using a spectrophotometer (Hitachi U-1100, Japan) at an absorption of 578 nm. For maintainance of anaerobic conditions of the medium and of the $Na_2Sx9H_2O$ solution, a nitrogen blanket of approx. 0.1 bar was applied to each storage vessel. The concentration of methane in the off-gas was between 60% and 90%.

## 6. Standard Fermentation with cell growth phase in 10 L scale

Cultivation of *M. marburgensis* was performed in standard medium in a 10 L bioreactor type Biostat® Cplus (Sartorius Stedim Biotech GmbH, Göttingen, Germany) at 65°C. For inoculation, a 50 mL suspension of a living culture of any strain, for example of *M. marburgensis,* was anaerobically transferred per litre of medium into the bioreactor using anaerobic procedures as described elsewhere (Sowers, Schreier *et al.*, 1995). Anaerobic 500 mM $Na_2Sx9H_2O$ was used as sulphur source and was fed continuously via peristaltic pump at a flow rate of 7.0 mL/h to 14.0 mL/h in the reaction vessel of the bioreactor.

The pH value was measured via pH probe (Mettler-Toledo GmbH, Greifensee, Switzerland) and kept constant at 6.8using 1 M $(NH_4)_2CO_3$ as base in order to compensate for acidification of the medium during growth of *M. marburgensis.* Oxidation reduction potential (ORP) was measured via redox probe (Mettler-Toledo GmbH, Greifensee, Switzerland). The individual gas flow of $H_2$ and $CO_2$ (Air Liquide, Paris, France) was controlled via mass flow controllers (Brooks Instrument, Matfield, USA). Off-gas was detected via individually serially applied gas analyzer systems (BlueSens gas sensor GmbH, Herten, Germany) for $H_2$, $CO_2$ and $CH_4$, respectively. The total gassing rate ($H_2$ and $CO_2$) was controlled by mass flow controllers. The in-flow rates of the gases were regulated depending on the determined partial pressure ratio of hydrogen to carbon dioxide.

In the initial cell growth phase, the partial pressure ratio of hydrogen to carbon dioxide was maintained in the range from 0.5:1 to 4:1 (parts hydrogen : parts carbon dioxide). Within this range from 0.5:1 to 4:1, the partial pressure ratio may fluctuate. In the cell growth phase, the fermentation, and therefore also the settings of the bioreactor, was started in batch cultivation mode with an ORP value below -300 mV. In a subsequent methane production phase, the partial pressure ratio of hydrogen to carbon dioxide was set to be maintained at a value of 5:1 or higher (parts hydrogen : parts carbon dioxide) and the fermentation mode was switched to continuous culture mode which was used for long term stable operation. The ORP value was kept below -350 mV. For a continuous culture of *M. marburgensis,* the medium feed supply was controlled gravimetrically via PID controller to maintain a constant feed rate.

Biomass was determined as dry weight using standard drying procedures and gravimetric determination of preweighted glass tubes. Optical density was measured using a spectrophotometer (Hitachi U-1100, Japan) at absorption of 578 nm. For maintainance of anaerobic conditions of the medium and of the $Na_2Sx9H_2O$ solution, a nitrogen blanket of approx. 0.1 bar was applied to each storage vessel.

The volume inside the reaction vessel was held constant via a harvest pump connected to an immersion pipe. Therefore, the reactor weight was kept constant over a feedback regulation on the harvest pump. The concentration of methane in the off-gas was between 60% and 90%.

### 7. Fermentation conditions depending on the species of the methanogenic microorganism

The above described fermentation conditions are principally the same for all methanogenic microorganisms of the invention, in particular for methanogenic archaea of the species *Methanosarcinia barkeri, Methanothermobacter marburgensis,* such as for example DSM 2133 (DSMZ, Braunschweig, Germany), *Methanobacterium thermoautotrophicus, Methanocaldococcus jannaschii, Methanothermobacter thermoautotrophicus, Methanococcus maripaludis,* or mixtures thereof. Table 1 summarizes fermentation conditions which have to be adjusted according to the species of the methanogenic microorganism used in the particular experiment. Further information on general fermentation conditions can be obtained from DSMZ, Braunschweig, Germany.

Table 1: Species dependent fermentation parameters as they can also be obtained from DSMZ, Braunschweig, Germany.

| Strain species | Range | | Preferred value | |
|---|---|---|---|---|
| | T [°C] | pH | T [°C] | pH |
| *Methanothermobacter marburgensis* | 63 - 68 | 6.7 - 7.0 | 65 | 6.8 |
| *Methanothermobacter thermautotrophicus, Methanobacterium thermoautotrophicus* | 63 - 68 | 6.7-8.0 | 65 | 6.8 |
| *Methanosarcina barkeri* | 35 - 37 | 6.5 - 6.8 | 36 | 6.7 |
| *Methanobrevibacter arboriphilicus* | 35 - 38 | 6.7 - 7.0 | 37 | 6.8 |
| *Methanocaldococcus jannaschii* | 80 - 87 | 5.0 - 6.3 | 85 | 6 |

### 8. Volumetric productivity of methane as a function of partial pressure ratio of hydrogen to carbon dioxide

Three 5 L batch cultivations of *M. marburgensis* with different partial pressure ratios of $H_2/CO_2$ were performed. The partial pressure ratio $H_2/CO_2$ was varied for each experiment by adjusting the in-gas flows according to the partial pressure determined by off-gas concentrations and head pressure measurement.

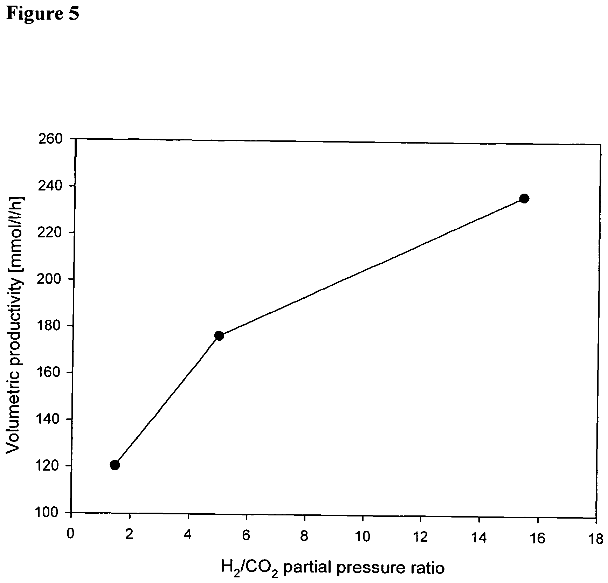

The volumetric productivity of the methanogenic microorganisms at the end of the exponential phase was taken as indicator for the cell's performance depending on the $H_2/CO_2$ partial pressure ratios during fermentation. The results as presented in Figure 5 show, that the higher the partial pressure ratio of $H_2/CO_2$ the higher the volumetric productivity of methane.

**9. Biomass yield per mol carbon dioxide as a function of the partial pressure ratio of hydrogen to carbon dioxide**

Three 5 L batch cultivations of *M. marburgensis* with different partial pressure ratios of $H_2/CO_2$ were performed. The partial pressure ratios $H_2/CO_2$ were varied for each experiment by adjusting the in-gas flows according to the partial pressure determined by off-gas concentrations and head pressure measurement.

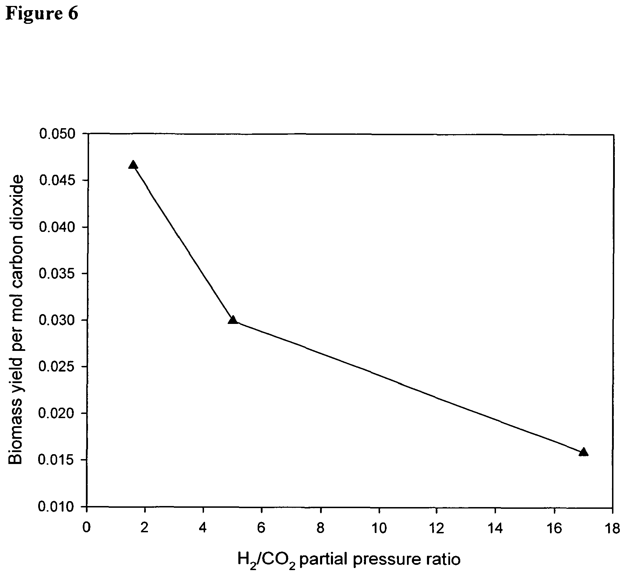

The biomass yield per mol carbon dioxide of the culture of methanogenic microorganisms was determined during the exponential phase. "Exponential phase" or "exponential cell growth phase" in the sense of the invention denotes the phase of exponential cell growth which is not to be mixed up with the cell growth phase of the invention characterized by a specific $H_2/CO_2$ partial pressure ratio. Several experiments with different $H_2/CO_2$ partial pressure ratios were performed. The results presented in Figure 6 show that the lower the partial pressure ratio of $H_2/CO_2$ the higher the biomass yield per mol carbon dioxide.

**10. Volumetric productivity of methane as a function of time and switch from cell growth phase to methane production phase**

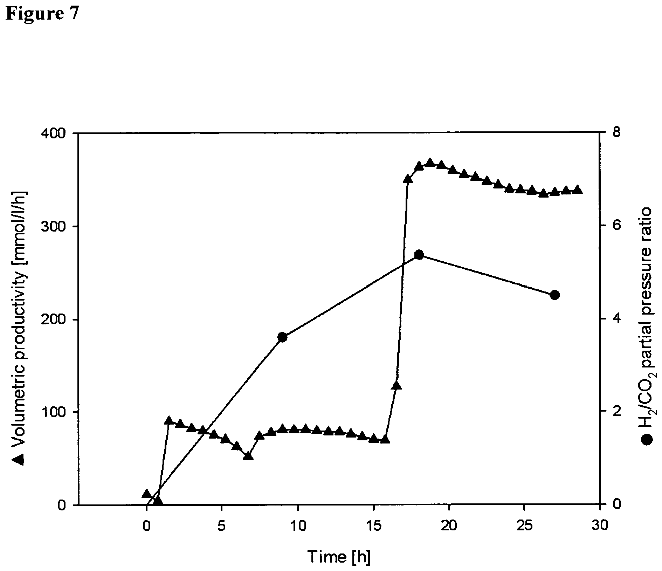

A 3 L batch culture of *M. marburgensis* with an initial partial pressure ratio of $H_2/CO_2$ of 2:1 was performed. After about 15 h, the culture was switched to continuous cultivation mode, with a constant feed rate of a dilution rate (which is the reciprocal of the residence time of the medium in the bioreactor and is derived by the feed rate divided by the working/culture volume of the reaction vessel inside the bioreactor) of 0.05 L medium/h. At the same time, the partial pressure ratio of $H_2/CO_2$ was switched from a ratio of $2\pm1{:}1$ to a ratio above 5:1 (parts hydrogen : parts carbon dioxide), by adjusting the in-gas flow of both gases according to the partial pressure determined by off-gas concentrations and head pressure measurement as described above. Figure 7 shows the trajectory of the volumetric productivity over time. Although shortly a high methane production can be observed prior to the shift to higher partial pressure ratios at the end of the cell growth phase, however, advantageous sustained and continuous high methane production can only be achieved by switching to a higher partial pressure ratio, i.e. to a $H_2/CO_2$ partial pressure ratio of 5:1 or higher.

**11. Biomass yield per mol carbon dioxide during switch from cell growth phase to methane production phase**

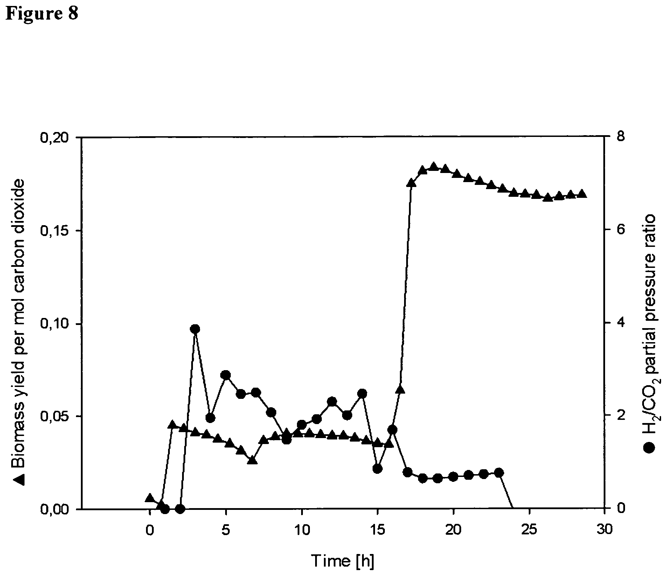

A 3 L batch culture of *M. marburgensis* with an initial partial pressure ratio of $H_2/CO_2$ of $2\pm1{:}1$ (parts hydrogen : parts carbon dioxide) was fermented in batch cultivation mode. After about 15 h, the culture was switched to continuous cultivation mode, with a constant feed rate of a dilution rate of 0.05 L medium/h. At the same time, the partial pressure ratio of $H_2/CO_2$ was switched from a ratio of $2\pm1{:}1$ to a ratio above 5:1 (parts hydrogen : parts carbon dioxide), by adjusting the in-gas flow of both gases according to the partial pressure determined by off-gas concentrations and head pressure measurement as described above. Samples were taken every hour in order to determine biomass concentration. The results presented in Figure 8 demonstrate, that the biomass yield per mol carbon dioxide with a partial pressure ratio of $H_2/CO_2$ of $2\pm1{:}1$ (parts hydrogen : parts carbon dioxide) was significantly higher than with a partial pressure ratio above 5:1 (parts hydrogen : parts carbon dioxide).

**12. Process stability**

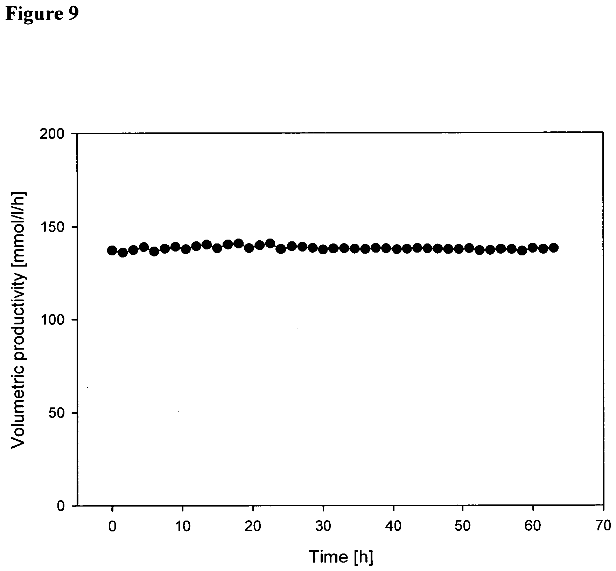

A cell culture of *M. marburgensis* in continuous mode with a constant feed rate of the medium of a dilution rate of 0.05 L/h was set up. Different partial pressure ratios of $H_2/CO_2$ were adjusted by varying the in-gas flow of $H_2/CO_2$ according to the partial pressure determined by off-gas concentrations and head pressure measurement. Figure 9 presents the volumetric productivity trajectory in continuous mode. The partial pressure ratio of $H_2/CO_2$ in the off-gas was maintained around $18\pm1{:}1$ (parts hydrogen : parts carbon dioxide). All cultures showed robust performance over several days and weeks. In the concrete example shown in Figure 9, the methanogenic microorganisms showed stable performance over 42 days. Around 130 mmol to 145 mmol of methane were stably produced per litre cell culture volume per hour over the whole period of time.

**Claims**

**1.** A method for storing energy in the form of methane comprising at least the following steps:

    a) generating electric energy using a renewable and/or non-renewable energy source;
    b) using at least partially the electric energy of step a) for the electrolysis of water and/or brine, thereby producing hydrogen and/or oxygen; and
    c) using at least partially the hydrogen of step b) and carbon dioxide, and optionally partially the electric energy of step a), for producing methane by methanogenic microorganisms in a reaction vessel, comprising contacting the methanogenic microorganisms with an in-gas feed comprising at least said hydrogen and carbon dioxide,

wherein step c) comprises at least one methane production phase, wherein in the at least one methane production phase the ratio of the partial pressure of hydrogen to the partial pressure of carbon dioxide inside the reaction vessel is maintained at 5:1 or higher (parts hydrogen : parts carbon dioxide).

2. The method according to claim 1 further comprising step d), wherein at least the oxygen of step b) is used for producing at least partially the carbon dioxide used in step c) by oxygen enriched combustion or gasification.

3. The method according to claim 1 or 2, wherein the electric energy of step a) is generated using a renewable energy source.

4. The method according to any of the preceding claims, wherein the renewable energy source comprises solar energy, wind power, wave power, tidal power, water or hydro power, geothermal energy, biomass and biofuel combustion, or combinations thereof.

5. The method according to any of the preceding claims, wherein the methanogenic microorganisms are methanogenic archaea.

6. The method according to claim 5, wherein the methanogenic archaea is selected from the group consisting of *Methanosarcinia barkeri, Methanobacterium thermoautotrophicus, Methanothermobacter thermoautotrophicus, Methanococcus maripaludis, Methanothermobacter marburgensis, Methanocaldococcus jannaschii,* and mixtures thereof.

7. The method according to any of the preceding claims, wherein step c) further comprises at least one cell growth phase, wherein in the at least one cell growth phase the ratio of the partial pressure of hydrogen to the partial pressure of carbon dioxide inside the reaction vessel is maintained at a ratio different from the ratio in the at least one methane production phase.


**Patentansprüche**

1. Ein Verfahren zur Speicherung von Energie in der Form von Methan, umfassend mindestens die folgenden Schritte:

   a) Erzeugen von elektrischer Energie unter Verwendung einer erneuerbaren und/oder nicht-erneuerbaren Energiequelle;
   b) Verwenden von mindestens teilweise der elektrischen Energie von Schritt a) für die Elektrolyse von Wasser und/oder Salzlake, dadurch Produzieren von Wasserstoff und/oder Sauerstoff; und
   c) Verwenden von mindestens teilweise dem Wasserstoff von Schritt b) und Kohlenstoffdioxid, und optional teilweise der elektrischen Energie von Schritt a), zum Herstellen von Methan durch methanogene Mikroorganismen in einem Reaktionsgefäß, umfassend das In-Kontakt-Bringen der methanogenen Mikroorganismen mit einer Eintrittsgaszufuhr, umfassend mindestens den Wasserstoff und das Kohlenstoffdioxid,

   wobei Schritt c) mindestens eine Methanproduktionsphase umfasst, wobei in der mindestens einen Methanproduktionsphase das Verhältnis des Partialdrucks von Wasserstoff zu dem Partialdruck von Kohlenstoffdioxid innerhalb des Reaktionsgefäßes bei 5:1 oder höher (Teile Wasserstoff : Teile Kohlenstoffdioxid) gehalten wird.

2. Das Verfahren gemäß Anspruch 1 weiterhin umfassend Schritt d), wobei mindestens der Sauerstoff von Schritt b) zum Erzeugen von mindestens teilweise dem Kohlenstoffdioxid, welches in Schritt c) verwendet wird, durch sauerstoffangereicherte Verbrennung oder Vergasung verwendet wird.

3. Das Verfahren gemäß Anspruch 1 oder 2, wobei die elektrische Energie von Schritt a) unter Verwendung einer erneuerbaren Energiequelle erzeugt wird.

4. Das Verfahren gemäß einem der vorangehenden Ansprüche, wobei die erneuerbare Energiequelle Solarenergie, Windenergie, Wellenenergie, Gezeitenenergie, Wasser- oder Hydroenergie, Erdwärme, Biomasse- und Biokraftstoff verbrennung, oder Kombinationen davon, umfasst.

**EP 2 675 904 B2**

5. Das Verfahren gemäß einem der vorangehenden Ansprüche, wobei die methanogenen Mikroorganismen methanogene Archaea sind.

6. Das Verfahren gemäß Anspruch 5, wobei die methanogenen Archaea ausgewählt sind aus der Gruppe bestehend aus *Methanosarcinia barkeri, Methanobacterium thermoautotrophicus, Methanothermobacter thermoautotrophicus, Methanococcus maripaludis, Methanothermobacter marburgensis, Methanocaldococcus jannaschii* und Gemischen davon.

7. Das Verfahren gemäß einem der vorangehenden Ansprüche, wobei Schritt c) zusätzlich mindestens eine Zellwachstumsphase umfasst, wobei in der mindestens einen Zellwachstumsphase das Verhältnis des Partialdrucks von Wasserstoff zu dem Partialdruck von Kohlenstoffdioxid innerhalb des Reaktionsgefäßes bei einem Verhältnis gehalten wird, das sich von dem Verhältnis in der mindestens einen Methanproduktionsphase unterscheidet.

**Revendications**

1. Procédé pour stocker de l'énergie sous forme de méthane comprenant au moins les étapes suivantes :

   a) la génération d'énergie électrique en utilisant une source d'énergie renouvelable et/ou non renouvelable ;
   b) l'utilisation, au moins partiellement, de l'énergie électrique de l'étape a) pour l'électrolyse d'eau et/ou de saumure, produisant ainsi de l'hydrogène et/ou de l'oxygène ; et
   c) l'utilisation, au moins partiellement, de l'hydrogène de l'étape b) et de dioxyde de carbone, et optionnellement partiellement, de l'énergie électrique de l'étape a), pour produire du méthane par des microorganismes méthanogènes dans une cuve de réaction, comprenant la mise en contact des microorganismes méthanogènes avec une alimentation en gaz comprenant au moins lesdits hydrogène et dioxyde de carbone,

   dans lequel l'étape c) comprend au moins une phase de production de méthane, dans lequel, dans l'au moins une phase de production de méthane, le rapport de la pression partielle d'hydrogène sur la pression partielle de dioxyde de carbone à l'intérieur de la cuve de réaction est maintenu à 5:1 ou plus (parts d'hydrogène:parts de dioxyde de carbone).

2. Procédé selon la revendication 1 comprenant en outre l'étape d), dans lequel au moins l'oxygène de l'étape b) est utilisé pour produire au moins partiellement le dioxyde de carbone utilisé à l'étape c) par combustion ou gazéification enrichie en oxygène.

3. Procédé selon la revendication 1 ou 2, dans lequel l'énergie électrique de l'étape a) est générée en utilisant une source d'énergie renouvelable.

4. Procédé selon l'une quelconque des revendications précédentes, dans lequel la source d'énergie renouvelable comprend l'énergie solaire, l'énergie éolienne, l'énergie houlomotrice, l'énergie marémotrice, l'énergie hydraulique, l'énergie géothermique, la combustion de biomasse et de biocarburant, ou des combinaisons de celles-ci.

5. Procédé selon l'une quelconque des revendications précédentes, dans lequel les microorganismes méthanogènes sont des archéobactéries méthanogènes.

6. Procédé selon la revendication 5, dans lequel les archéobactéries méthanogènes sont sélectionnées dans le groupe constitué de *Methanosarcinia barkeri, Methanobacterium thermoautotrophicus, Methanothermobacter thermoautotrophicus, Methanococcus maripaludis, Methanothermobacter marburgensis, Methanocaldococcusjannaschii,* et des mélanges de celles-ci.

7. Procédé selon l'une quelconque des revendications précédentes, dans lequel l'étape c) comprend en outre au moins une phase de croissance cellulaire, dans lequel, dans l'au moins une phase de croissance cellulaire, le rapport de la pression partielle d'hydrogène sur la pression partielle de dioxyde de carbone à l'intérieur de la cuve de réaction est maintenu à un rapport différent du rapport dans l'au moins une phase de production de méthane.

**Figure 1**

# Figure 2

**Figure 3**

**Figure 4**

**Figure 5**

# Figure 6

Figure 7

Figure 8

**Figure 9**

# Figure 10

## Figure 11

**Figure 12**

**EP 2 675 904 B2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20100269498 A
- DE 102007037672
- US 20090130734 A1
- WO 2008094282 A

### Non-patent literature cited in the description

- **STERNER ; SPECHT.** *Solarzeitalter,* 2010, vol. 1 (10), 51-58
- Water Treatment, Principles and Design. John Wiley & Sons, 2005
- **BAILEY, OLLIS.** Biochemical Engineering Fundamentals. McGraw-Hill Science, 1986